# EUROPEAN PATENT APPLICATION

(11) **EP 4 475 130 A1**
(43) Date of publication of application: **11.12.2024**
(21) Application number: 24179123.5
(22) Date of filing: 30.05.2024
(51) Int. Cl.: G16C 20/20, H01J 49/00, G16C 20/70

(54) **SUPPORT SYSTEMS FOR MASS SPECTROMETRY SCIENTIFIC INSTRUMENTS**

(30) Priority: 01.06.2023 US 202363505650 P
(71) Applicant: Thermo Fisher Scientific (Bremen) GmbH, 28199 Bremen (DE); MSAID GmbH, 85748 Munich (DE)
(72) Inventor: HENRICH, Christoph, 28199 Bremen (DE); FREJNO, Martin, 85748 Munich (DE); SCHMIDT, Tobias, 85748 Munich (DE); SEEFRIED, Florian, 85748 Munich (DE); FRITZEMEIER, Kai, 28199 Bremen (DE)
(74) Representative: Boult Wade Tennant LLP

(57) **Abstract**

Disclosed herein are scientific instrument support systems, as well as related methods, computing devices, and computer-readable media. For example, in some embodiments, a scientific instrument support apparatus including memory hardware configured to store instructions and processing hardware configured to execute the instructions. The instructions include loading a batch of raw spectrum files generated by a mass spectrometer, dividing the raw spectrum files into a first subset and a second subset, processing each of the first subset of raw spectrum files with a machine learning model to generate a first subset of spectrum match files, generating a screening list from the first subset of spectrum match files, and processing each of the second subset of raw spectrum files and the screening list with the machine learning model to generate a second subset of spectrum match files.

## Description

### Cross-Reference to Related Applications

This application claims the priority of U.S. Provisional Application 63/505,650, filed on June 1, 2023 entitled "SUPPORT SYSTEMS FOR MASS SPECTROMETRY SCIENTIFIC INSTRUMENTS", the entire disclosure of which is incorporated herein by reference in its entirety.

### Technical Field

Various embodiments relate generally, but not exclusively, to scientific instruments and scientific instrument support apparatuses, such as mass spectrometers and support apparatuses for mass spectrometers.

### Summary

Scientific instruments may include a complex arrangement of movable components, sensors, input and output ports, energy sources, and consumable components. Data generated by the sensors may be saved and processed by scientific instrument support apparatuses. For example, in a typical proteomics analysis run, mass spectrometers may generate thousands up to millions or billions of mass spectra for a single batch of protein samples. These mass spectra are typically stored as raw spectra files, containing all spectra belonging to one measurement. Each raw spectrum file may record the mass-to-charge ratios (m/z) and their corresponding intensities for each ion detected in the mass spectrometer. Raw spectrum files may contain many spectra, typically from a chromatography run. These raw spectrum files may serve as the starting point for proteomic data analysis. For example, the raw spectrum files may be analyzed using various computational techniques to identify and/or quantify the peptides and/or proteins that may be present in the batch of protein samples.

Some techniques include first processing a batch of raw spectrum files to generate an initial spectrum match file for each raw spectrum file. A batch of raw spectrum files may include a collection of data files from multiple measurements of the same sample or multiple measurements of multiple samples. Spectrum match files may include information such as probable peptide sequencies, protein identifications, and confidence scores for a corresponding raw spectrum file. The initial spectrum match files are then processed to generate screening lists or lists of entities of interest - such as inclusion and/or exclusion lists. The raw spectrum files are then reprocessed with the lists of entities of interest to generate a result file for each raw spectrum file. The result files are then analyzed to generate a results list. Using such techniques, the entire batch of raw spectrum files must be processed to generate initial spectrum match files and the entire batch of initial spectrum match files must be processed to generate the screening lists. The entire batch of raw spectrum files must then be re-processed with the inclusion and/or exclusion lists to generate result files, and the entire batch of result files must be processed to generate the results list. In some embodiments, the entire batch of initial result files must be reprocessed to generate a consensus report.

Given the large size of each batch of data (e.g., thousands or tens of thousands of files) and that the entire batch must be processed multiple times (typically twice), techniques such as the one previously described are computationally intensive - and it may be computationally infeasible to perform real-time or near-real-time data analysis using them. What is needed are optimized techniques that reduce the computational burden and increase computational throughput to allow for real-time or near-real-time data analysis.

One example provides a scientific instrument support apparatus including memory hardware configured to store instructions and processing hardware configured to execute the instructions. The instructions include loading a batch of raw spectrum files generated by a mass spectrometer, dividing the raw spectrum files into a first subset and a second subset, processing each of the first subset of raw spectrum files with a machine learning model to generate a first subset of spectrum match files, generating a screening list from the first subset of spectrum match files, and processing each of the second subset of raw spectrum files and the screening list with the machine learning model to generate a second subset of spectrum match files.

In other features, the instructions include generating a results list from the second subset of spectrum match files. In other features, the instructions include processing each of the first subset of raw spectrum files and the screening list with the machine learning model to generate an updated first subset of spectrum match files and generating a results list from the updated first subset of spectrum match files and the second subset of spectrum match files. In other features, the machine learning model is configured to generate each spectrum match file by preprocessing a selected raw spectrum file, loading a protein database, generating a test spectrum for each peptide in the protein database, and matching spectra in the preprocessed spectrum file with the generated test spectra and generating a score evaluating a closeness of each match. In other features, the machine learning model is configured to generate each spectrum file by determining whether the screening list is loaded and, in response to determining that the screening list is not loaded, discarding matched spectra having scores below a first threshold and saving remaining matched spectra to the spectrum match file.

In other features, the machine learning model is configured to generate each spectrum file by determining whether the screening list is loaded. In response to determining that the screening list is loaded, the machine learning model is configured to generate each spectrum file by determining whether the screening list includes an inclusion list, discarding matched spectra having scores below a first threshold and that are not on the inclusion list in response to determining that the screening list includes the inclusion list, determining whether the screening list includes an exclusion list, and discarding matched spectra on the exclusion list in response to determining that the screening list includes the exclusion list. The machine learning model is configured to generate each spectrum file by discarding matched spectra having scores below the first threshold and saving remaining matched spectra to the spectrum match file. In other features, generating the screening list from the first subset of spectrum match files includes parsing the first subset of spectrum match files to identify peptides present, calculating a frequency of appearance for each of the identified peptides, discarding identified peptides having a frequency of appearance below a second threshold, and adding the remaining identified peptides to an inclusion list.

In other features, generating the screening list from the first subset of spectrum match files includes generating filtered spectrums by removing peaks below an intensity threshold from spectrums of the first subset of spectrum match files, processing the filtered spectrums to identify peptides associated with the filtered spectrums, counting a number of occurrences of each identified peptide, and saving peptides having a number of occurrences below a third threshold to the exclusion list. In other features, preprocessing the selected raw spectrum file includes detecting peaks in a spectrum of the raw spectrum file, removing noise from the spectrum, applying a baseline correction to the spectrum, applying mass calibration to the spectrum, and applying deconvolution processing to the spectrum. In other features, the mass spectrometer generates raw spectrum files by ionizing a prepared sample, performing ion separation on the ionized sample, detecting separated ions, and generating a mass spectrum from the detected separated ions.

Other examples provide a computer-implemented method for scientific instrument support includes loading a batch of raw spectrum files generated by a mass spectrometer, dividing the raw spectrum files into a first subset and a second subset, processing each of the first subset of raw spectrum files with a machine learning model to generate a first subset of spectrum match files, generating a screening list from the first subset of spectrum match files, and processing each of the second subset of raw spectrum files and the screening list with the machine learning model to generate a second subset of spectrum match files.

In other features, the method includes generating a results list from the second subset of spectrum match files. In other features, the method includes processing each of the first subset of raw spectrum files and the screening list with the machine learning model to generate an updated first subset of spectrum match files and generating a results list from the updated first subset of spectrum match files and the second subset of spectrum match files. In other features, the machine learning model is configured to generate each spectrum match file by preprocessing a selected raw spectrum file, loading a protein database, generating a test spectrum for each peptide in the protein database, and matching spectra in the preprocessed spectrum file with the generated test spectra and generating a score evaluating a closeness of each match. In other features, the machine learning model is configured to generate each spectrum file by determining whether the screening list is loaded and, in response to determining that the screening list is not loaded, discarding matched spectra having scores below a first threshold and saving remaining matched spectra to the spectrum match file.

In other features, the machine learning model is configured to generate each spectrum file by determining whether the screening list is loaded. In response to determining that the screening list is loaded, the machine learning model is configured to generate each spectrum file by determining whether the screening list includes an inclusion list, discarding matched spectra having scores below a first threshold and that are not on the inclusion list in response to determining that the screening list includes the inclusion list, determining whether the screening list includes an exclusion list, and discarding matched spectra on the exclusion list in response to determining that the screening list includes the exclusion list. The machine learning model is configured to generate each spectrum file by discarding matched spectra having scores below the first threshold and saving remaining matched spectra to the spectrum match file. In other features, generating the screening list from the first subset of spectrum match files includes parsing the first subset of spectrum match files to identify peptides present, calculating a frequency of appearance for each of the identified peptides, discarding identified peptides having a frequency of appearance below a second threshold, and adding the remaining identified peptides to an inclusion list.

In other features, generating the screening list from the first subset of spectrum match files includes generating filtered spectrums by removing peaks below an intensity threshold from spectrums of the first subset of spectrum match files, processing the filtered spectrums to identify peptides associated with the filtered spectrums, counting a number of occurrences of each identified peptide, and saving peptides having a number of occurrences below a third threshold to the exclusion list. In other features, preprocessing the selected raw spectrum file includes detecting peaks in a spectrum of the raw spectrum file, removing noise from the spectrum, applying a baseline correction to the spectrum, applying mass calibration to the spectrum, and applying deconvolution processing to the spectrum. In other features, the mass spectrometer generates raw spectrum files by ionizing a prepared sample, performing ion separation on the ionized sample, detecting separated ions, and generating a mass spectrum from the detected separated ions.

In other features, one or more non-transitory computer-readable media includes instructions thereon that, when executed by one or more processing devices of a scientific instrument support apparatus, cause the scientific instrument support apparatus to perform the method.

According to some examples, a scientific instrument support apparatus includes first logic to receive a batch of raw data structures generated by a mass spectrometer and second logic to divide the batch of raw data structures into a first subset and a second subset, generate a first subset of processed data structures by providing each of the first subset of raw data structures to an artificial-intelligence-enabled data analysis system, parse the first subset of processed data structures to build a comparison list, and generate a second subset of processed data structures by providing each of the second subset of raw data structures and the comparison list to the artificial-intelligence-enabled data analysis system.

In other features, the mass spectrometer is configured to generate the raw data structures by ionizing a prepared sample, performing ion separation on the ionized sample, detecting separated ions, and generating a mass spectrum from the detected separated ions. In other features, the artificial-intelligence-enabled data analysis system is configured to preprocess a selected data structure, load a database, generate a test spectrum for each peptide in the database, and match spectra in the preprocessed data structure with the generated test spectra and generate a score evaluating a closeness of each match. In other features, the artificial-intelligence-enabled data analysis system is configured to determine whether the comparison list is loaded and, in response to determining that the comparison list is not loaded, discard matched spectra having scores below a first threshold, and save remaining matched spectra to the processed data structure.

In other features, the artificial-intelligence-enabled data analysis system is configured to determine whether the comparison list is loaded. In response to determining that the comparison list is loaded, the artificial-intelligence-enabled data analysis system is configured to determine whether the comparison list includes an inclusion list, discard matched spectra having scores below a first threshold and that are not on the inclusion list in response to determining that the comparison list includes the inclusion list, determine whether the comparison list includes an exclusion list, and discard matched spectra on the exclusion list in response to determining that the comparison list includes the exclusion list. The artificial-intelligence-enabled data analysis system is configured to discard matched spectra having scores below the first threshold and save remaining matched spectra to the processed data structure. In other features, preprocessing the selected data structure includes detecting peaks in a spectrum of the selected data structure, removing noise from the spectrum, applying a baseline correction to the spectrum, applying mass calibration to the spectrum, and applying deconvolution processing to the spectrum.

In other features, the second logic is configured to build the comparison list by parsing the first subset of processed data structures to identify peptides present, calculating a frequency of appearance for each of the identified peptides, discarding identified peptides having a frequency of appearance below a second threshold, and adding the remaining identified peptides to an inclusion list. In other features, the second logic is configured to build the comparison list by parsing the first subset of processed data structures to generate filtered spectrums by removing peaks below an intensity threshold, processing the filtered spectrums to identify peptides associated with the filtered spectrums, counting a number of occurrences of each identified peptide, and saving peptides having a number of occurrences below a third threshold to the exclusion list. In other features, the second logic is configured to generate an output list by processing the second subset of processed data structures. In other features, the second logic is configured to generate an updated first subset of processed data structures by providing each of the first subset of raw data structures and the comparison list to the artificial-intelligence-enabled data analysis system and generate an output list by processing the updated first subset of processed data structures and the second subset of processed data structures.

Other examples provide a method for scientific instrument support includes loading a batch of raw data structures generated by a mass spectrometer, dividing the batch of raw data structures into a first subset and a second subset, generating a first subset of processed data structures by providing each of the first subset of raw data structures to an artificial-intelligence-enabled data analysis system, parsing the first subset of processed data structures to build a comparison list, and generating a second subset of processed data structures by providing each of the second subset of raw data structures and the comparison list to the artificial-intelligence-enabled data analysis system.

In other features, the mass spectrometer is configured to generate the raw data structures by ionizing a prepared sample, performing ion separation on the ionized sample, detecting separated ions, and generating a mass spectrum from the detected separated ions. In other features, the artificial-intelligence-enabled data analysis system is configured to preprocess a selected data structure, load a database, generate a test spectrum for each peptide in the database, match spectra in the preprocessed data structure with the generated test spectra, and generate a score evaluating a closeness of each match. In other features, the artificial-intelligence-enabled data analysis system is configured to determine whether the comparison list is loaded and, in response to determining that the comparison list is not loaded, discarding matched spectra having scores below a first threshold and saving remaining matched spectra to the processed data structure.

In other features, the artificial-intelligence-enabled data analysis system is configured to determine whether the comparison list is loaded. In response to determining that the comparison list is loaded, the artificial-intelligence-enabled data analysis system is configured to determine whether the comparison list includes an inclusion list, discard matched spectra having scores below a first threshold and that are not on the inclusion list in response to determining that the comparison list includes the inclusion list, determine whether the comparison list includes an exclusion list, and discard matched spectra on the exclusion list in response to determining that the comparison list includes the exclusion list. The artificial-intelligence-enabled data analysis system is configured to discard matched spectra having scores below the first threshold and save remaining matched spectra to the processed data structure.

In other feature, preprocessing the selected data structure includes detecting peaks in a spectrum of the selected data structure, removing noise from the spectrum, applying a baseline correction to the spectrum, applying mass calibration to the spectrum, and applying deconvolution processing to the spectrum. In other features, parsing the first subset of processed data structures to build the comparison list includes parsing the first subset of processed data structures to identify peptides present, calculating a frequency of appearance for each of the identified peptides, discarding identified peptides having a frequency of appearance below a second threshold, and adding the remaining identified peptides to an inclusion list.

In other features, parsing the first subset of processed data structures to build the comparison list includes parsing the first subset of processed data structures to generate filtered spectrums by removing peaks below an intensity threshold, processing the filtered spectrums to identify peptides associated with the filtered spectrums, counting a number of occurrences of each identified peptide, and saving peptides having a number of occurrences below a third threshold to the exclusion list. In other features, the method includes generating an output list by processing the second subset of processed data structures. In other features, the method includes generating an updated first subset of processed data structures by providing each of the first subset of raw data structures and the comparison list to the artificial-intelligence-enabled data analysis system and generating an output list by processing the updated first subset of processed data structures and the second subset of processed data structures.

In other features, one or more non-transitory computer-readable media includes instructions thereon that, when executed by one or more processing devices of a scientific instrument support apparatus, cause the scientific instrument support apparatus to perform the method.

Some examples include a method for scientific instrument support including receiving a first set of mass spectrometry data, processing the first set of mass spectrometry data to generate a database of identified entities, receiving a second set of mass spectrometry data, and processing the second set of mass spectrometry data to identify and/or quantitate entities based on the database of identified entities.

In other features, the first set of mass spectrometry data and the second set of mass spectrometry data are generated using a same data acquisition method. In other features, the data acquisition method is a data independent acquisition method. In other features, the data acquisition method is a data dependent acquisition method. In other features, processing the first set of mass spectrometry data to generate the database of identified entities includes comparing ion spectra from the first set of mass spectrometry data to a reference database. In other features, processing the first set of mass spectrometry data to generate the database of identified entities includes adding entities from the first set of mass spectrometry data that meet a minimum quality criterion to the database of identified entities. In other features, the minimum quality criterion is set according to at least one of a threshold, false detection rate, or spectral match score.

In other features, the database of identified entities includes peptide sequences. In other features, the database of identified entities includes peptide identifications. In other features, the database of identified entities includes mass spectra. In other features, the database of identified entities includes precursor ion information. In other features, the precursor ion information includes mass information. In other features, the precursor ion information includes mass-to-charge ratios. In other features, the precursor ion information includes mass-to-charge windows. In other features, the method includes processing the first set of mass spectrometry data to identify and/or quantitate entities based on the database of identified entities. In other features, the method includes processing the second set of mass spectrometry data to identify and/or quantitate entities based on the database of identified entities includes comparing ion spectra from the second set of mass spectrometry data with entries in the database of identified entities.

In other features, the method includes processing the second set of mass spectrometry data to identify and/or quantitate entities based on the database of identified entities includes comparing fragmentation spectra from the second set of mass spectrometry data with entries in the database of identified entities. In other features, the method includes processing the second set of mass spectrometry data to identify and/or quantitate entities based on the database of identified entities includes searching the second set of mass spectrometry data for entities in the database of identified entities. In other features, searching the second set of mass spectrometry data for entities in the database of identified entities includes searching the database of identified entities for at least one of precursor information or retention time information. In other features, the method includes processing at least some of the second set of mass spectrometry data to extend the database of identified entities.

In other features, processing at least some of the second set of mass spectrometry data to extend the database of identified entities includes re-searching already processed members of the first and second sets of mass spectrometry data to receive further identification and/or quantification information. In other features, processing at least some of the second set of mass spectrometry data to extend the database of identified entities is stopped in response to a growth rate of the database of identified entities falling below a second threshold. In other features, the second threshold is an average of less than 10 addition entries per member of the second set of mass spectrometry data. In other features, the second threshold is an average of less than 1 addition entries per member of the second set of mass spectrometry data. In other features, the second threshold is an average of less than 0.1 addition entries per member of the second set of mass spectrometry data. In other features, the second threshold is an average of less than 0.01 addition entries per member of the second set of mass spectrometry data.

In other features, members of the first set of mass spectrometry data are selected to have a higher concentration than members of the second set of mass spectrometry data. In other features, scientific instrument support apparatus includes memory hardware configured to store instructions and processing hardware configured to execute the instructions, which when executed by the processing hardware causes the scientific instrument support apparatus to perform the method.

In other features, one or more non-transitory computer-readable media includes instructions thereon that, when executed by one or more processing devices of a scientific instrument support apparatus, cause the scientific instrument support apparatus to perform the method.

Examples include a method for scientific instrument support includes receiving a first set of mass spectrometry files representing one or more samples, analyzing each spectrum file of the first set of mass spectrometry data with a selected machine learning model from a first set of machine learning models to generate initial results, analyzing the initial results to generate a screening list, receiving one or more raw spectrum files from a second set of mass spectrometry data, analyzing each of the one or more raw spectrum files from the second set of mass spectrometry data at a selected machine learning model from a second set of machine learning models to generate result files, and saving the result files to a data store.

In other features, the selected machine learning model from the first set of machine learning models is the same as the selected machine learning model from the second set of machine learning models. In other features, the selected machine learning model from the first set of machine learning models is different from the selected machine learning model from the second set of machine learning models. In other features, the selected machine learning model from the first set of machine learning models and the selected machine learning model from the second set of machine learning models includes a database search engine. In other features, the database search engine is a peptide search engine.

In other features, analyzing the initial results to generate the screening list includes merging high-confidence identifications from all searches into one screening list of identified entities for a given experimental setup. In other features, scientific instrument support apparatus includes memory hardware configured to store instructions and processing hardware configured to execute the instructions, which when executed by the processing hardware causes the scientific instrument support apparatus to perform the method.

In other features, one or more non-transitory computer-readable media includes instructions thereon that, when executed by one or more processing devices of a scientific instrument support apparatus, cause the scientific instrument support apparatus to perform the method.

A method for scientific instrument support includes receiving a first subset of a set of mass spectrometry data, receiving a first screening list, processing the first subset of mass spectrometry data and the first screening list at a first database search engine to generate a second screening list, receiving a second subset of the set of mass spectrometry data, and providing each file of the second subset of mass spectrometry data and a target screening list to a second database search engine to generate a result file for each file of the second subset of mass spectrometry data, the target screening list being based on the second screening list.

In other features, the second screening list is provided to the second database search engine as the target screening list. In other features, the target screening list is generated by merging the first screening list and the second screening list. In other features, the set of mass spectrometry data includes data from one or more connected studies. In other features, the set of mass spectrometry data includes at least one of mass data, intensity data, a retention time, ion mobility data, a physico-chemical property, and a location on a spatially arranged sample. In other features, elements of the set of mass spectrometry data are related by at least one of a similarity of samples and a similarity of data acquisition methods. In other features, the first screening list is formatted in a FASTA format. In other features, processing the first subset of mass spectrometry data and the first screening list at the first database search engine to generate the second screening list includes selecting entities according to criteria.

In other features, the entities include proteins or peptides. In other features, selecting entities according to criteria includes determining that each entities passes or fails a quality control test and adding the entity to a database of identified entities in response to determining that each entity passes the quality control test. In other features, the quality control test includes at least one of selecting entities based on a false discovery rate, determining whether entities meet or exceed a spectral quality threshold, determining whether entities have at least a number of peaks in common with a reference, and determining whether entities meet or exceed a minimum number of occurrences in the subset. In other features, the quality control test includes ranking entities according to a percolator machine learning model and separating true positive entity identifications from incorrect entity identifications.

In other features, each entity is represented by at least one of an entity identifier, a protein sequence, a peptide sequence, one or more masses from a mass spectrometry (MS) spectrometer, one or more masses from a tandem mass spectrometry (MS/MS) spectrometer, an intensity value, a physico-chemical property, a retention time, or an ion mobility. In other features, providing each file of the second subset of mass spectrometry data and the target screening list to the second database search engine to generate the result file for each file of the second subset of mass spectrometry data includes at least one of excluding any entities not present in the target screening list from further processing and including any entities present in the target screening list for further processing.

In other features, providing each file of the second subset of mass spectrometry data and the target screening list to the second database search engine to generate the result file for each file of the second subset of mass spectrometry data includes comparing mass spectrometry data from each file of the second subset to library spectra data. In other features, providing each file of the second subset of mass spectrometry data and the target screening list to the second database search engine to generate the result file for each file of the second subset of mass spectrometry data includes mass spectrometry data from each file of the second subset to synthetic spectra created based on entities present in the target screening list.

In other features, mass spectrometry data from each file of the second subset includes at least one of mass data, intensity data, retention time data, and ion mobility data. In other features, the first database search engine and the second database search engine apply same processing toolchains. In other features, the first database search engine and the second database search engine apply different processing toolchains. In other features, the first database search engine matches entities from the first subset of mass spectrometry data with first reference entities based on a first criterion, the second database search engine matches entities from the second subset of mass spectrometry data with second reference entities based on a second criterion, and the first criterion requires a greater match than the second criterion.

In other features, the first criterion includes matching entities based on at least one of fragments, mass deviation, retention time, and physico-chemical properties. In other features, the second criterion includes matching entities based on at least one of fragments, mass deviation, retention time, and physico-chemical properties. In other features, the second database search engine is configured to output an aligned database of identifications per sample. In other features, the second database search engine is configured to perform further processing steps by calculating a quantitation value. In other features, the second database search engine is configured to calculate the quantitation value based on relative intensities within a sample. In other features, the second database search engine is configured to calculate the quantitation value based on relative intensities across samples.

In other features, the second database search engine is configured to calculate the quantitation value from signal intensities across multiple neighboring mass spectra. In other features, the second database search engine is configured to calculate the quantitation value from spectral contribution factors across multiple neighboring mass spectra. In other features, the second database search engine is configured to calculate the quantitation value using unlabeled calibration substances. In other features, the second database search engine is configured to calculate the quantitation value using labeled calibration substances. In other features, labels of the labeled calibration substances include at least one of mass tags and isotopic labels.

In other features, the second database search engine is configured to determine occurrences across at least one of the set of mass spectrometry data, the first subset of the mass spectrometry data, the second subset of the mass spectrometry data, further subsets of the mass spectrometry data, and a third subset including the first subset and one or more additional elements of the set of mass spectrometry data. In other features, the second database search engine is configured to compare occurrences across at least one of the set of mass spectrometry data, the first subset of the mass spectrometry data, the second subset of the mass spectrometry data, further subsets of the mass spectrometry data, and a third subset including the first subset and one or more additional elements of the set of mass spectrometry data.

In other features, the second database search engine is configured to determine quantitation comparisons across at least one of the set of mass spectrometry data, the first subset of the mass spectrometry data, the second subset of the mass spectrometry data, further subsets of the mass spectrometry data, and a third subset including the first subset and one or more additional elements of the set of mass spectrometry data. In other features, the second database search engine is configured to output a database of identifications and quantitations across the set of mass spectrometry data. In other features, the second database search engine is configured to output a database of identifications and quantitations across a portion of set of mass spectrometry data. In other features, the method further includes outputting the at least one result file to a graphical user interface displayed on a screen. The graphical user interface is configured to allow a user or other data system to interrogate the at least one result file for at least one of: (i) significant differences between samples, (ii) a presence of substances within one or more samples, and (iii) an absence of substances within one or more samples.

In other features, a scientific instrument support apparatus includes memory hardware configured to store instructions and processing hardware configured to execute the instructions, which when executed by the processing hardware causes the scientific instrument support apparatus to perform the method. In other features, one or more non-transitory computer-readable media having instructions thereon that, when executed by one or more processing devices of a scientific instrument support apparatus, cause the scientific instrument support apparatus to perform the method.

### Brief Description of the Drawings

Embodiments will be readily understood by the following detailed description in conjunction with the accompanying drawings. To facilitate this description, like reference numerals designate like structural elements. Embodiments are illustrated by way of example, not by way of limitation, in the figures of the accompanying drawings.
FIG. 1 is a block diagram of a scientific instrument support module for performing support operations.
FIGS. 2A and 2B are flowcharts of a first example process for processing sensor data generated by a scientific instrument.
FIGS. 3A, 3B, and 3C are flowcharts of a second example process for processing sensor data generated by a scientific instrument.
FIG. 4 is a flowchart of an example process for generating raw spectrum files from a batch of samples using a mass spectrometer.
FIGS. 5A and 5B are flowcharts of an example process for processing raw spectrum files to generate result files.
FIG. 6 is a flowchart of an example process for generating screening lists from spectrum match files.
FIG. 7 is a flowchart of an example process for generating screening lists from spectrum match files.
FIG. 8 is a flowchart of an example process for generating results lists from spectrum match files.
FIG. 9 is a flowchart of an example process for preprocessing raw spectrum files.
FIG. 10 depicts an example graphical user interface that may be used in the performance of scientific instrument support methods.
FIG. 11 is a block diagram of a computing device that may perform scientific instrument support methods.
FIG. 12 is a block diagram of an example scientific instrument support system in which scientific instrument support methods may be performed.
FIG. 13 is a block diagram of an example implementation of a portion of the first example process of FIGS. 2A and 2B.
FIG. 14 is a block diagram of an example implementation of a portion of the second example process of FIGS. 3A, 3B, and 3C.
FIG. 15 is a block diagram of an example implementation of a portion of the second example process of FIGS. 3A, 3B, and 3C.
FIG. 16 is a block diagram of an example implementation of a portion of the second example process of FIGS. 3A, 3B, and 3C.
FIG. 17 is a block diagram of an example implementation of a portion of the second example process of FIGS. 3A, 3B, and 3C.

### Detailed Description

Disclosed herein are scientific instrument support systems, as well as related methods, computing devices, and computer-readable media. For example, in some embodiments, a scientific instrument support apparatus including memory hardware configured to store instructions and processing hardware configured to execute the instructions. The instructions include loading a batch of raw spectrum files generated by a mass spectrometer, dividing the raw spectrum files into a first subset and a second subset, processing each of the first subset of raw spectrum files with a machine learning model to generate a first subset of spectrum match files, generating a screening list from the first subset of spectrum match files, and processing each of the second subset of raw spectrum files and the screening list with the machine learning model to generate a second subset of spectrum match files.

The scientific instrument support embodiments disclosed herein may achieve improved performance relative to conventional approaches. For example, in proteomics, mass spectrometry instruments are used to generate mass spectra of biological samples (such as protein samples). Each mass spectrum may be represented as a histogram plot of relative intensities versus mass-to-charge ratios (m/z) of the chemical compounds present in the biological samples. Thus, when used in proteomics, each mass spectrum may represent a chemical component of a peptide (or multiple peptides) - the building blocks of proteins. Peptides are generated by digestion during preparation of the biological samples before they are analyzed. Typically, the combined mass spectra generated from a biological sample may be analyzed using various techniques to identify the peptides present in the sample.

A variety of problems exist with conventional mass spectrometry techniques (and associated data synthesis and analysis techniques). For example, each biological sample is typically chemically decomposed before being analyzed by a mass spectrometer. Thus - in some examples - each individual sample can only be analyzed once. This often results in a high level of run-to-run variance between samples. This variance can arise because (i) the biological sample does not decompose perfectly into its constituent peptides, (ii) the biological sample and/or the solvent used to decompose the proteins are contaminated, (iii) the biological sample itself is imperfect - for example, there may be compositional and/or structural variances between different samples of the same protein, and/or (iv) there is instrumentation error introduced by the mass spectrometer. Because of these problems, mass spectra generated from an individual sample cannot be considered reliable indicators of a protein's composition. Mass spectra generated from each individual sample may tend to be missing data and/or contain excess data (e.g., because of noise introduced by contaminants or instrumentation error). Thus, to build a reliable picture of a protein's chemical composition, mass spectra from large batches of samples are typically analyzed using statistical methods or other algorithms to (i) fill in missing data and/or (ii) eliminate noise.

On such analysis technique is the match-between-runs technique. Generally, match-between-runs techniques may (i) detect peptide features in individual runs (such as chromatographic peaks corresponding to peptide ions), (ii) characterize the detected features (for example, according to their retention time [RT], mass-to-charge ratio [m/z], and/or intensity), (iii) identify peptides by comparing peptide features (such as their experimental spectra) to theoretical or measured spectra generated by protein databases, (iv) performing retention time alignment to account for variability in retention times between runs, (v) matching peptide features across multiple runs, (vi) applying a false detection rate (FDR) threshold to control the rate of false-positive identifications, (vii) performing intensity normalization operations to ensure the intensities of matched features are comparable across all runs, and/or (viii) performing data integration and analysis operations by integrating the aligned and matched peptide features into a single dataset.

To further improve peptide identification, reduce missing values, enhance reproducibility, and improve the overall performance of match-between-runs techniques, inclusion and/or exclusion lists may be used during the match-between-runs process. For example, inclusion and/or exclusion lists may be used during peptide identification and/or peptide matching phases to prioritize peptide ions in the inclusion list and/or remove noise from data. In conventional approaches, (i) all spectra in a dataset are processed using database search algorithms to generate matches, (ii) the matches for the entire dataset are processed to generate inclusion and/or exclusion lists, and (iii) all spectra in the entire dataset are then re-processed with the generated inclusion and/or exclusion lists to generate updated matches.

In typical mass spectrometry analysis runs, many thousands - or tens of thousands - of raw spectrum files may be generated for a batch of protein samples. These thousands or tens of thousands of raw spectrum files must be (i) processed, (ii) analyzed to generate inclusion and/or exclusion lists, and (iii) re-processed with the inclusion and/or exclusion lists. The massive computational requirements associated with processing mass spectrometry datasets using conventional techniques makes real-time or near-real-time processing nearly. Accordingly, new computational techniques that improve the computational throughput of mass spectrometry systems are needed to allow for real-time or near-real-time results.

The embodiments disclosed herein thus provide improvements to scientific instrument technology (e.g., improvements in the computer technology supporting such scientific instruments, among other improvements). As previously discussed, the embodiments disclosed herein may achieve higher-computational throughput relative to conventional approaches. Various ones of the embodiments disclosed herein may improve upon conventional approaches to achieve the technical advantages of improving computational throughput and allowing mass spectrometry data to be processed in real time or near-real time. Such technical advantages are not achievable by routine and conventional approaches, and all users of systems including such embodiments may benefit from these advantages (e.g., by assisting the user in the performance of a technical task, such as generating data using a mass spectrometer and processing the generated data, by means of a guided human-machine interaction process). The technical features of the embodiments disclosed herein are thus decidedly unconventional in the field of mass spectrometry, as are the combinations of the features of the embodiments disclosed herein. As discussed further herein, various aspects of the embodiments disclosed herein may improve the functionality of a computer itself; for example, by improving the throughput of the computer. The computational and user interface features disclosed herein do not only involve the collection and comparison of information but apply new analytical and technical techniques to change the operation of data processing and analysis pipelines in mass spectrometry. The present disclosure thus introduces functionality that neither a conventional computing device, nor a human, could perform.

Accordingly, the embodiments of the present disclosure may serve any of a number of technical purposes, such as controlling a specific technical system or process; determining from measurements how to control a machine; separation of sources in a mixed signal; optimizing load distribution in a computer network; providing estimates and confidence intervals for biological samples; simulating the behavior of a technical item or process; deriving a genotype estimate; reducing the amount of sensor data to be processed; and/or providing a faster processing of sensor data. The embodiments disclosed herein thus provide improvements to mass spectrometry technology (e.g., improvements in the computer technology supporting mass spectrometry, among other improvements).

In the following detailed description, reference is made to the accompanying drawings that form a part hereof wherein like numerals designate like parts throughout, and in which is shown, by way of illustration, embodiments that may be practiced. It is to be understood that other embodiments may be utilized, and structural or logical changes may be made, without departing from the scope of the present disclosure. Therefore, the following detailed description is not to be taken in a limiting sense.

Various operations may be described as multiple discrete actions or operations in turn, in a manner that is most helpful in understanding the subject matter disclosed herein. However, the order of description should not be construed as to imply that these operations are necessarily order dependent. In particular, these operations may not be performed in the order of presentation. Operations described may be performed in a different order from the described embodiment. Various additional operations may be performed, and/or described operations may be omitted in additional embodiments.

For the purposes of the present disclosure, the phrases "A and/or B" and "A or B" mean (A), (B), or (A and B). For the purposes of the present disclosure, the phrases "A, B, and/or C" and "A, B, or C" mean (A), (B), (C), (A and B), (A and C), (B and C), or (A, B, and C). Although some elements may be referred to in the singular (e.g., "a processing device"), any appropriate elements may be represented by multiple instances of that element, and vice versa. For example, a set of operations described as performed by a processing device may be implemented with different ones of the operations performed by different processing devices.

The description uses the phrases "an embodiment," "various embodiments," and "some embodiments," each of which may refer to one or more of the same or different embodiments. Furthermore, the terms "comprising," "including," "having," and the like, as used with respect to embodiments of the present disclosure, are synonymous. When used to describe a range of dimensions, the phrase "between X and Y" represents a range that includes X and Y. As used herein, an "apparatus" may refer to any individual device, collection of devices, part of a device, or collections of parts of devices. The drawings are not necessarily to scale.

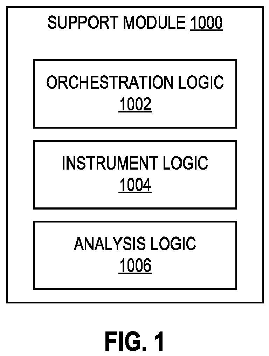

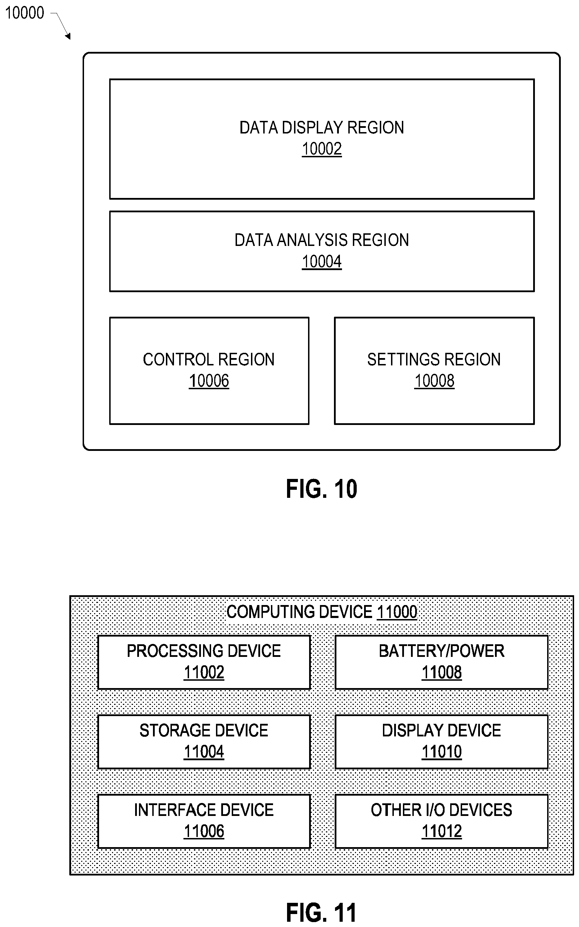

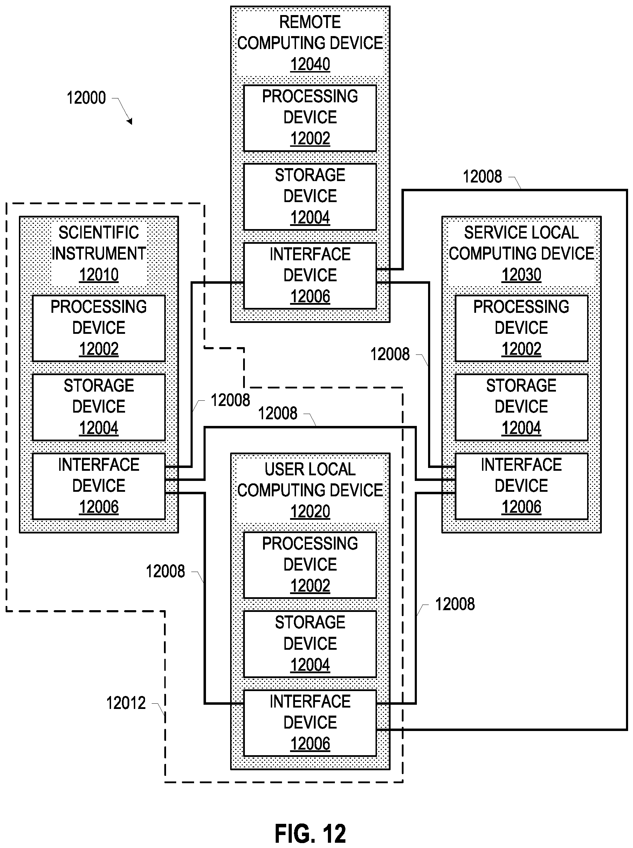

FIG. 1 is a block diagram of a scientific instrument support module 1000 for performing support operations, in accordance with various embodiments. The scientific instrument support module 1000 may be implemented by circuitry (e.g., including electrical and/or optical components), such as a programmed computing device. The logic of the scientific instrument support module 1000 may be included in a single computing device or may be distributed across multiple computing devices that are in communication with each other as appropriate. Examples of computing devices that may, singly or in combination, implement the scientific instrument support module 1000 are discussed herein with reference to the computing device 11000 of FIG. 11, and examples of systems of interconnected computing devices, in which the scientific instrument support module 1000 may be implemented across one or more of the computing devices, is discussed herein with reference to the scientific instrument support system 12000 of FIG. 12.

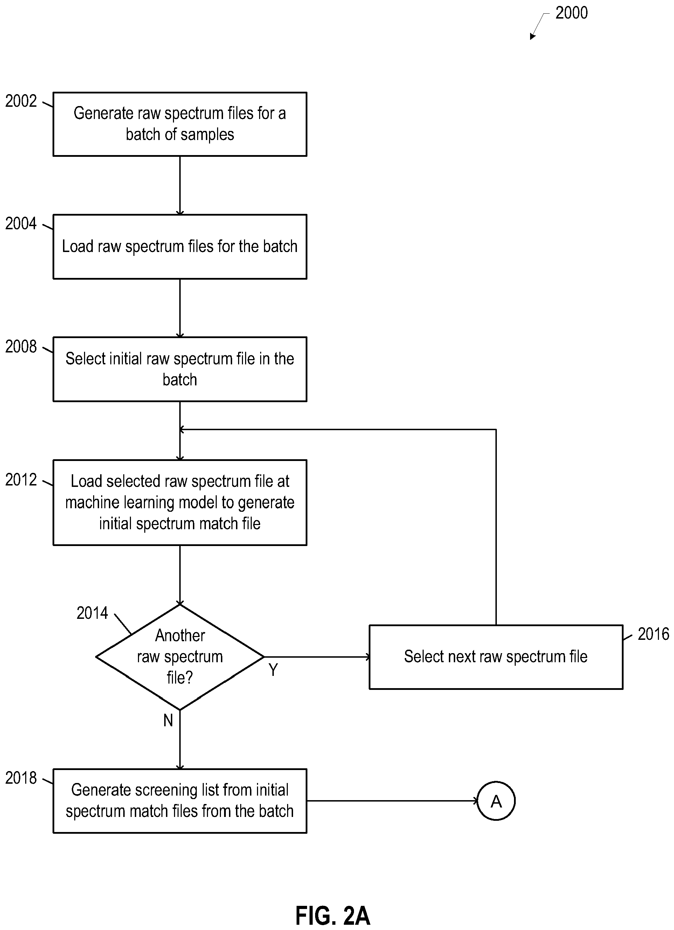

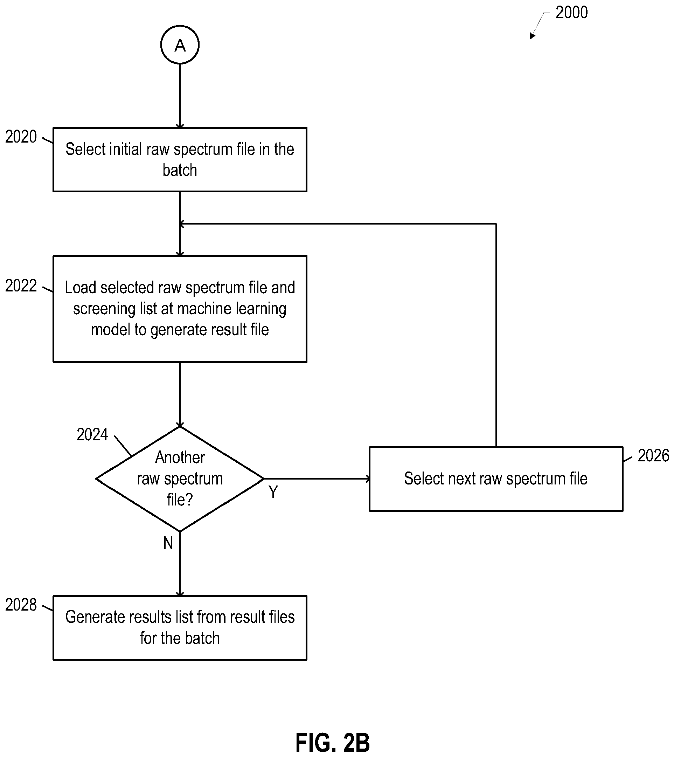

The scientific instrument support module 1000 may include first logic - which may be referred to herein as orchestration logic 1002, second logic - which may be referred to herein as instrument logic 1004, and third logic - which may be referred to herein as analysis logic 1006. As used herein, the term "logic" may include an apparatus that is to perform a set of operations associated with the logic. For example, any of the logic elements included in the support module 1000 may be implemented by one or more computing devices programmed with instructions to cause one or more processing devices of the computing devices to perform the associated set of operations (e.g., collectively as a group or set of one or processing devices). In some embodiments, a logic element may include one or more non-transitory computer-readable media having instructions thereon that, when executed by one or more processing devices of one or more computing devices, cause the one or more computing devices to perform the associated set of operations. As used herein, the term "module" may refer to a collection of one or more logic elements that, together, perform a function associated with the module. Different ones of the logic elements in a module may take the same form or may take different forms. For example, some logic in a module may be implemented by a programmed general-purpose processing device, while other logic in a module may be implemented by an application-specific integrated circuit (ASIC). In other examples, different ones of the logic elements in a module may be associated with different sets of instructions executed by one or more processing devices. A module may not include all of the logic elements depicted in the associated drawing; for example, a module may include a subset of the logic elements depicted in the associated drawing when that module is to perform a subset of the operations discussed herein with reference to that module. Additional functionality of the orchestration logic 1002, instrument logic 1004, and/or analysis logic 1006 will be described further on in this specification with reference to FIGS. 2A-9.

FIGS. 2A-9 are flowcharts of example processes 2000-9000 for performing support operations, in accordance with various embodiments. Although the operations of the processes 2000-9000 may be illustrated with reference to particular embodiments disclosed herein (e.g., the scientific instrument support modules 1000 discussed herein with reference to FIG. 1, the GUI 10000 discussed herein with reference to FIG. 10, the computing devices 11000 discussed herein with reference to FIG. 11, and/or the scientific instrument support system 12000 discussed herein with reference to FIG. 12), the processes 2000-9000 may be used in any suitable setting to perform any suitable support operations. Operations are illustrated once each and in a particular order in FIGS. 2A-9, but the operations may be reordered and/or repeated as desired and appropriate (e.g., different operations performed may be performed in parallel, as suitable).

FIGS. 2A-2B are flowcharts of a first example process 2000 for processing sensor data (such as mass spectra data) generated by a scientific instrument (such as a mass spectrometer). At 2002, the orchestration logic 1002 and/or the instrument logic 1004 may generate raw spectrum files for a batch of samples. For example, the orchestration logic 1002 may generate a user interface with user interface elements - such as a data display region, a data analysis region, a control region, and a settings region. The user may command the scientific instrument - such as the mass spectrometer - to generate raw spectrum files for a batch of samples using the control region. In response to the user selecting one or more selectable commands on the user interface, the orchestration logic 1002 may command the instrument logic 1004 to send signals to the mass spectrometer for the mass spectrometer to generate the raw spectrum files. In various implementations, the raw spectrum files are generated according to data dependent acquisition (DDA) methods. In example DDA methods, the mass spectrometer selectively isolates and fragments precursor ions based on their abundance in a given scan. For example, the mass spectrometer may first perform a full scan of all ions in a sample to provide a spectrum of all ions present (sorted according to their mass-to-charge ratios [m/z]). The mass spectrometer then selects the most intense ions from the spectrum as precursor ions (these ions should correspond to the most abundant ions). Each selected precursor ion is then isolated and fragmented - for example, by collision-induced dissociation (CID) or higher-energy C-trap dissociation (HCD) - to create smaller product ions. The product ions are then analyzed in a second mass spectrometry scan, which generates a fragmentation spectrum for each precursor ion.

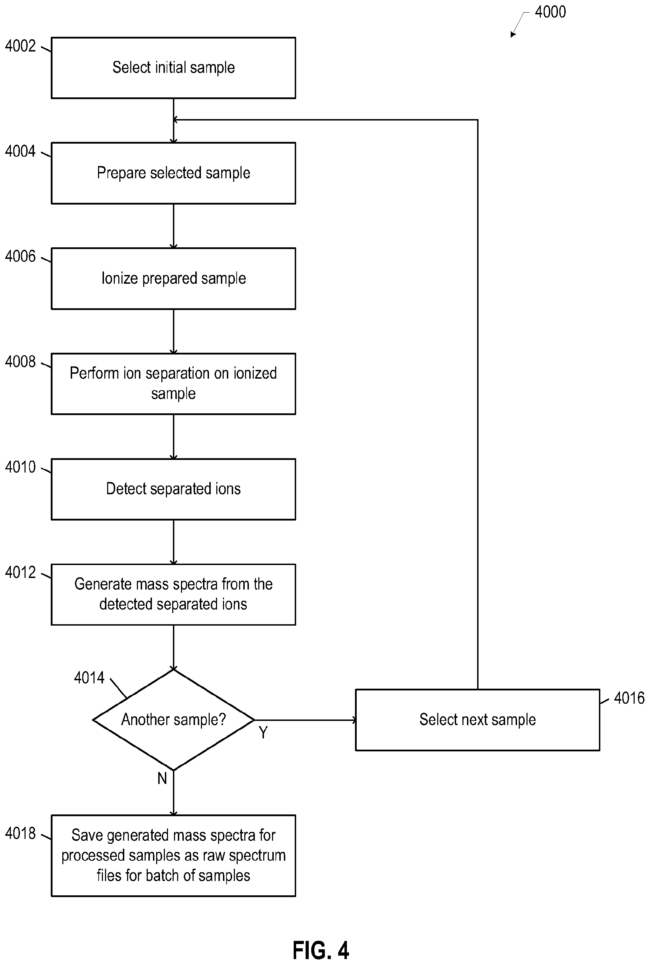

In some examples, the raw spectrum files are generated according to data independent acquisition methods (DIA). In contrast to DDA methods, DIA methods fragment all ions within a certain mass-to-charge ratio (m/z) range (regardless of their abundance). The mass spectrometer can then generate a fragmentation spectrum for each fragmented ion. Additional details associated with generating raw spectrum files for the batch of samples will be described further on in this specification with reference to FIG. 4.

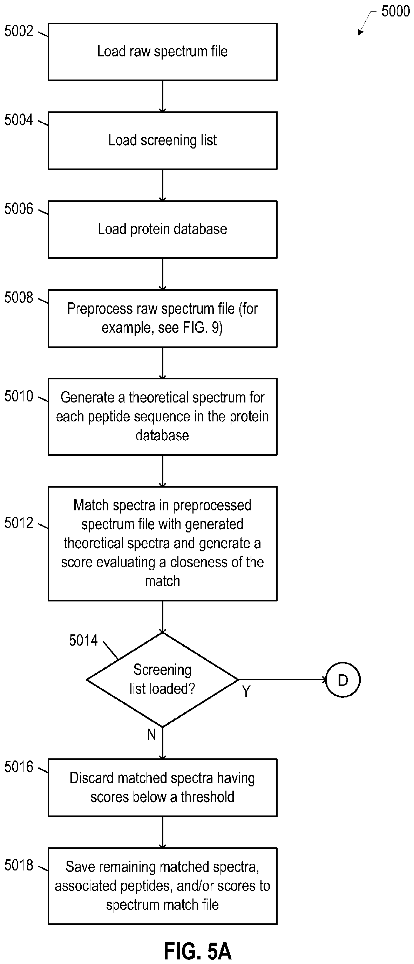

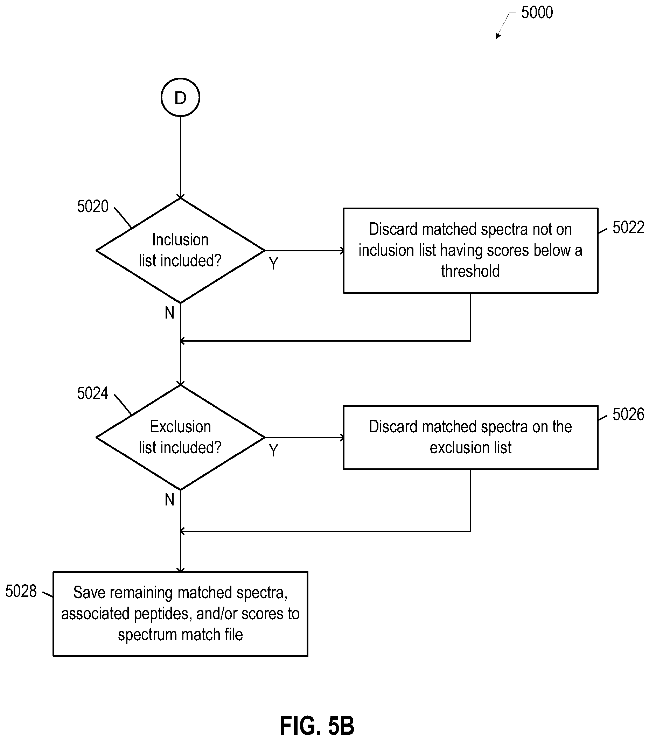

At 2004, the analysis logic 1006 loads the raw spectrum files generated for the batch of samples. For example, the user may select one or more user interface elements in the control region for the support module 1000 to begin data processing operations. In response to the user selecting the one or more user interface elements, the orchestration logic 1002 may command the analysis logic 1006 to retrieve the raw spectrum files from the instrument logic 1004 and load the raw spectrum files. At 2006, the analysis logic 1006 selects the initial raw spectrum file in the batch. At 2012, the analysis logic 1006 loads the selected raw spectrum file at a machine learning model - such as a database search engine - to generate an initial spectrum match file from the selected raw spectrum file. In various implementations, the database search engine may be an artificial-intelligence-enabled database search engine. Suitable examples of database search engines include SEQUEST software developed by the University of Washington, Mascot software developed by Matrix Science, Prosit software developed by the Technical University of Munich, X! Tandem software developed by The Global Proteome Machine Organization, Andromeda software - which is integrated with the MaxQuant software package developed by the Max-Planck-Institute of Biochemistry, the Open Mass Spectrometry Search Algorithm software developed by the National Institute of Health, Comet software developed by the University of Washington, MS-GF+software developed by the Pacific Northwest National Laboratory, PEAKS^{®} software developed by Bioinformatics Solutions Inc., SpectraST software developed by the Institute for Systems Biology, Byonic^{™} software developed by Protein Metrics, CHIMERYS^{®} software developed by MSAID GmbH, and/or Thermo Scientific^{™} Proteome Discoverer^{™} software, Thermo Scientific^{™} Orbitrap^{™}, and/or Thermo Scientific^{™} Q Exactive^{™} software developed by Thermo Fisher Scientific Inc. Additional details associated with generating the initial spectrum match file will be described further on in this specification with reference to FIGS. 5A and 5B.

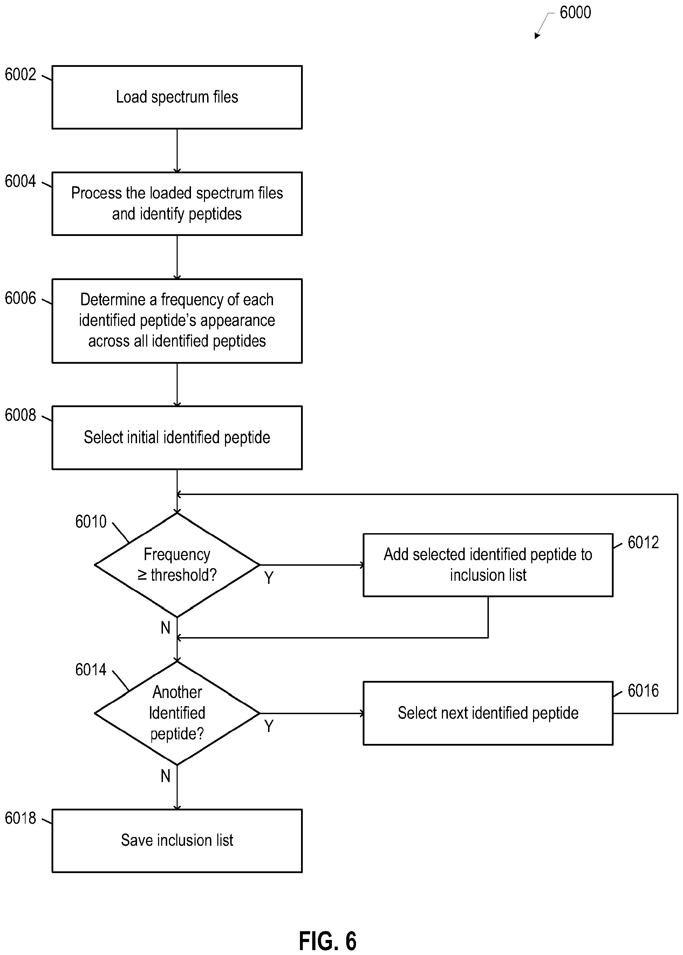

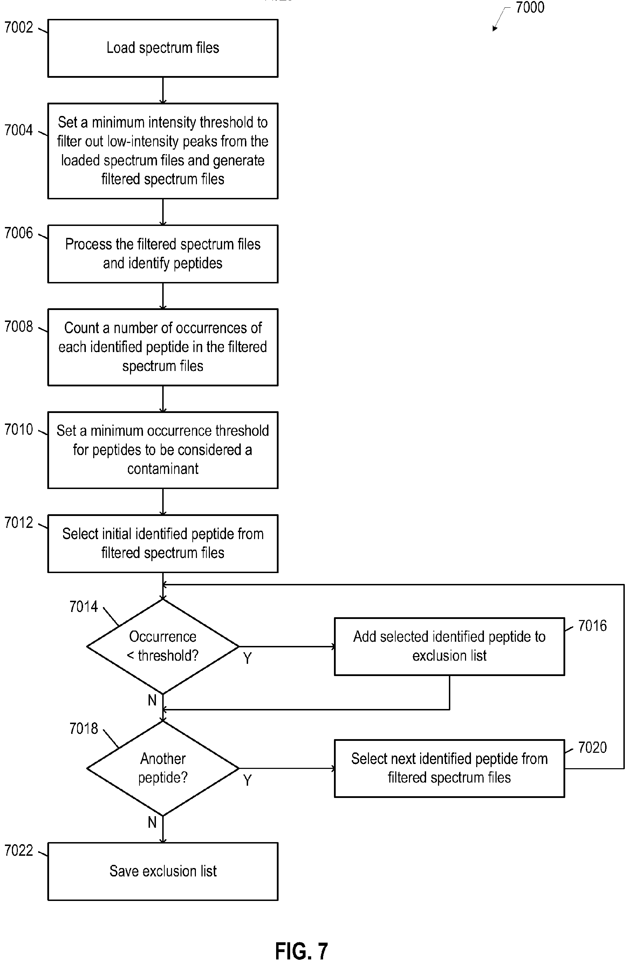

At 2014, the analysis logic 1006 determines whether another raw spectrum file that has not yet been processed at 2012 is present in the batch. In response to the analysis logic 1006 determining that another unprocessed spectrum file is present in the batch ("YES" at decision block 2014), the analysis logic 1006 selects the next raw spectrum file at 2016 and loads the selected raw spectrum file at the machine learning model to generate a corresponding initial raw spectrum match file at from the selected raw spectrum file at 2012. In response to the analysis logic 1006 determining that another unprocessed spectrum file is not present in the batch ("NO" at decision block 2014), the analysis logic 1006 generates a screening list from the initial spectrum match files of the batch at 2018. In some embodiments, the screening list may include entities of interest (such as peptides of interest). In some implementations analysis logic 1006 generates a database of identified entities instead of the screening list. Additional details associated with generating the screening list will be described further on in this specification with reference to FIGS. 6 and 7. At 2020, the analysis logic 1006 again selects the initial raw spectrum file in the batch. At 2022, the analysis logic 1006 loads the selected raw spectrum file and the screening list - such as the screening list generated at 2018 - at the machine learning model to generate a result file (such as an updated or refined spectrum match file) from the selected raw spectrum file and the screening list. In various implementations, the machine learning model may include any of the database search engines previously described with reference to 2012. Additional details associated with generating the result file will be described further on in this specification with reference to FIGS. 5A and 5B.

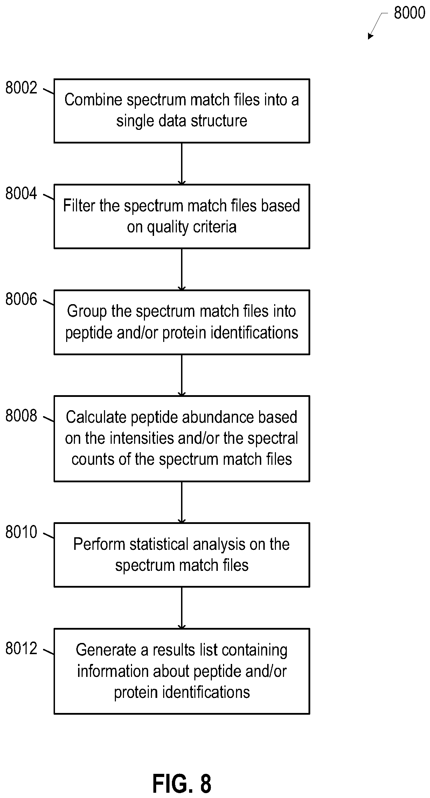

At 2024, the analysis logic 1006 determines whether another raw spectrum file that has not yet been processed at 2022 is present in the batch. In response to the analysis logic 1006 determining that another unprocessed spectrum file is present in the batch ("YES" at decision block 2024), the analysis logic 1006 selects the next raw spectrum file at 2026 and loads the selected raw spectrum file at the machine learning model to generate a corresponding result file from the selected raw spectrum file at 2022. In response to the analysis logic 1006 determining that another unprocessed spectrum file is not present in the batch ("NO" at decision block 2024), the analysis logic 1006 generates a results list from result files for the batch at 2028. Additional details associated with generating the results list will be described further on in this specification with reference to FIG. 8. In various implementations, the user interface may display the generated results to the user via the data display region and/or data analysis region.

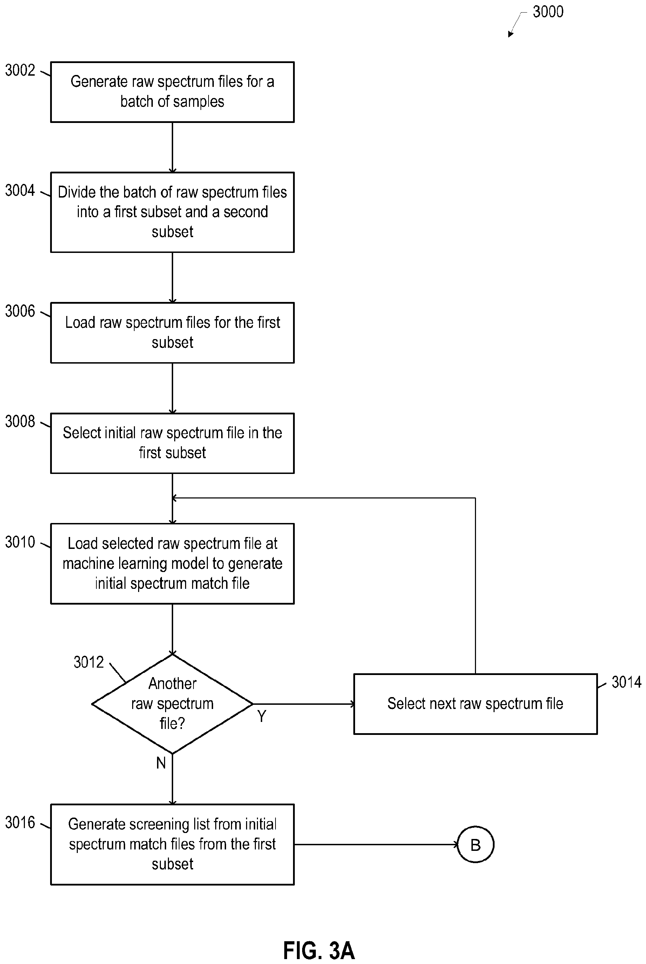

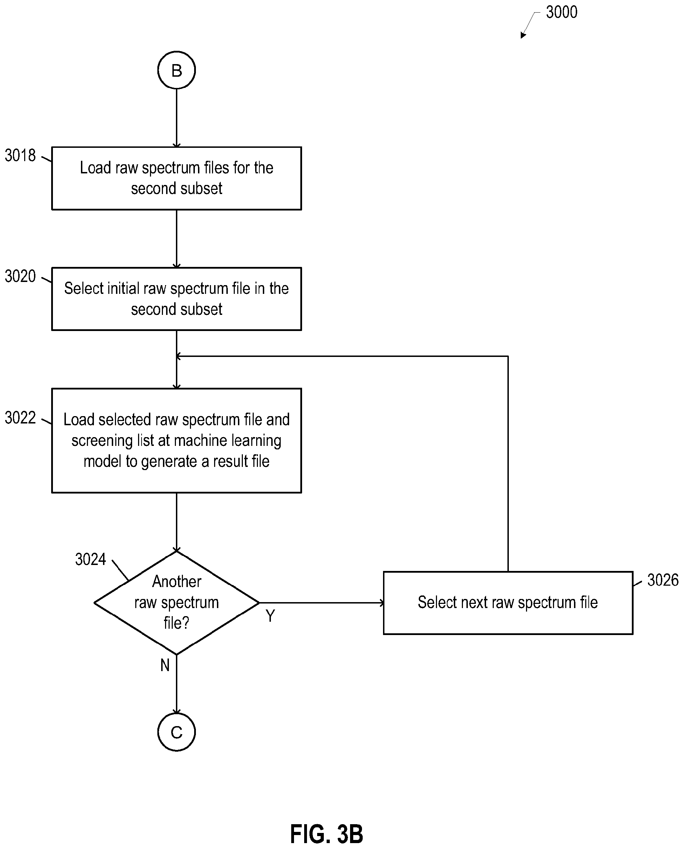

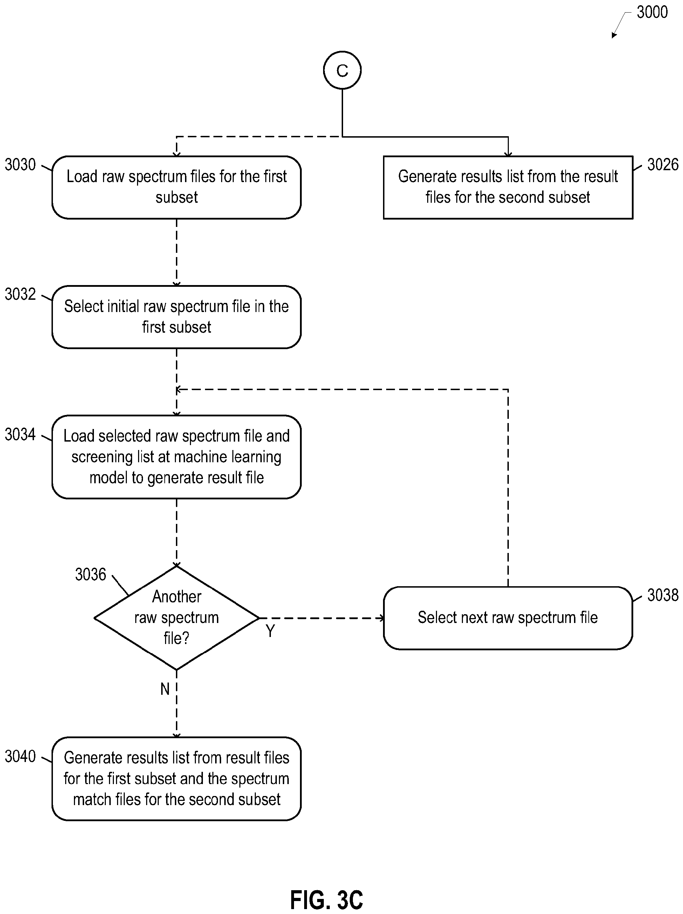

FIGS. 3A-3C are flowcharts of a second example process for processing sensor data (such as mass spectra data) generated by a scientific instrument (such as a mass spectrometer) using optimized techniques that improve computational throughput and allow for real-time or near-real-time data processing. At 3002, the orchestration logic 1002 generates raw spectrum files for a batch of samples. In various implementations, the raw spectrum files may be generated as previously described with reference to 2002. Additional details associated with generating raw spectrum files for the batch of samples will be described further on in this specification with reference to FIG. 4. At 3004, the analysis logic 1006 loads the raw spectrum files for the batch and divides the raw spectrum files into a first subset and a second subset. In various implementations, the raw spectrum files of the first subset and the second subset are generated according to the same data acquisition method. In various implementations, the analysis logic 1006 may load the raw spectrum files as previously described with reference to 2004. In some examples, the first subset and the second subset may be defined as percentages of the total batch. In some embodiments, the first subset may be about 10% of the batch and the second subset may be about 90% of the batch. In various implementations, the first subset may be about 20% of the batch and the second subset may be about 80% of the batch. In some examples, the first subset may be about 25% of the batch and the second subset may be about 75% of the batch. In some implementations, the first subset may be about 33% of the batch and the second subset may be about 67% of the batch. In example embodiments, the first subset may be about 50% of the batch and the second subset may be about 50% of the batch. In various implementations, the first subset includes a defined number of raw spectrum files from the batch. For example, the first subsets includes anywhere from one raw spectrum file to about 100 raw spectrum files from the batch. In some examples, the first subset is generated or selected to have a high probability of containing relevant raw spectrum files (for example, members of the first subset may have a higher concentration than members of the second subset, or samples may be pooled).

At 3006, the analysis logic 1006 loads the raw spectrum files for the first subset. At 3008, the analysis logic 1006 selects the initial raw spectrum file in the first subset. At 3010, the analysis logic 1006 loads the selected raw spectrum file at the machine learning model to generate an initial spectrum match file from the selected raw spectrum file. In some examples, the initial spectrum match file may be generated as previously described with reference to 2012. Additional details associated with generating raw spectrum files will be described later on in this specification with reference to FIGS. 5A and 5B. In various implementations, the analysis logic 1006 provides the selected raw spectrum file and a search space file to the machine learning model. The search space file may define a search space for the machine learning model. For example, in embodiments where the machine learning model includes a database search engine, the search space file may include a database of protein sequences. The database of protein sequences may include primary sequence data for proteins that the database search engine uses to define a complex search space that can be explored to match experimental spectra from the raw spectrum file with theoretical spectra derived from the database. In various implementations, the search space file may include a FASTA file.

At 3012, the analysis logic 1006 determines whether another raw spectrum file that has not yet been processed at 3010 is present in the first subset. In response to the analysis logic 1006 determining that another unprocessed raw spectrum file is present in the first subset ("YES" at decision block 3012), the analysis logic 1006 selects the next raw spectrum file at 3014 and loads the selected raw spectrum file at the machine learning model to generate a corresponding initial raw spectrum file from the selected raw spectrum file at 3010. In response to the analysis logic 1006 determining that another unprocessed raw spectrum file is not present in the first subset ("NO" at decision block 3012), the analysis logic 1006 generates a screening list from the initial spectrum match files for the first subset at 3016. In various implementations, the screening list may be generated as previously described with reference to 2018. In some examples, the analysis logic 1006 generates a database of identified entities instead of or in addition to the screening list. Additional details associated with generating the screening list will be described further on in this specification with reference to FIGS. 6 and 7.

At 3018, the analysis logic 1006 loads raw spectrum files for the second subset. At 3020, the analysis logic 1006 selects the initial raw spectrum file in the second subset. At 3022, the analysis logic 1006 loads the selected raw spectrum file and the screening list generated at 3016 at the machine learning model to generate a result file. Additional details associated with generating the result file will be described further on in this specification with reference to FIGS. 5A and 5B. At 3024, the analysis logic 1006 determines whether another raw spectrum file that has not yet been processed at 3022 is present in the second subset. In response to determining that another unprocessed raw spectrum file is present in the second subset ("YES" at decision block 3024), the analysis logic 1006 selects the next raw spectrum file in the second subset at 3026 and loads the selected raw spectrum file and screening list at the machine learning model to generate a result file (such as an updated or refined spectrum match file) from the selected raw spectrum file at 3022. In response to determining that another unprocessed raw spectrum file is not present in the second subset ("NO" at decision block 3024), the process 3000 proceeds either to 3028 or 3030.

In various implementations, the process 3000 proceeds from 3024 to 3028. At 3028, the analysis logic 1006 generates a results list from the result files for the second subset. Additional details associated with generating the results list will be discussed further on in this specification with reference to FIG. 8. In various implementations, the generated results list may be displayed to the user via the data display region and/or data analysis region of the user interface.

In some examples, the process 3000 proceeds from 3024 to 3030. At 3030, the analysis logic 1006 loads raw spectrum files for the first subset. At 3032, the analysis logic 1006 selects an initial raw spectrum file in the first subset. At 3034, the analysis logic 1006 loads the selected raw spectrum file and screening list generated at 3022 at the machine learning model to generate an result file. Additional details associated with generating the result file will be described further on in this specification with reference to FIGS. 5A and 5B. At 3036, the analysis logic 1006 determines whether another raw spectrum file that has not yet been processed at 3034 is present in the first subset. In response to determining that another unprocessed raw spectrum file is present in the first subset ("YES" at decision block 3036), the analysis logic 1006 selects the next raw spectrum file in the first subset at 3038 and loads the selected raw spectrum file and screening list at the machine learning model to generate a result file at 3034. In response to determining that another unprocessed raw spectrum file is not present in the first subset ("NO" at decision block 3036), the analysis logic 1006 generates a results list from result files for the first subset and spectrum match files for the second subset. Additional details associated with generating the results list will be described further on in this specification with reference to FIG. 8. In various implementations, the generated results list may be displayed to the user via the data display region and/or data analysis region of the user interface.

The example process 3000 may offer a variety of technical benefits not realized by other methods. For example, the process 3000 may generate a screening list at 3016 after processing only the raw spectrum files of the first subset. By contrast, techniques such as those described in example process 2000 generate a screening list only after processing raw spectrum files for the entire batch. By generating the screening list after processing raw spectrum files of only a subset- which may be substantially smaller than the full batch, the example process 3000 dramatically reduces the amount of computation required, thus improving the efficiency and throughput of the support module 1000. By improving the efficiency and throughput, the example process 3000 allows the support module 1000 to achieve real-time or near-real-time processing of mass spectra from scientific instruments - technical effects that may not be achieved by techniques such as example process 2000.

FIG. 4 is a flowchart of an example process 4000 for generating raw spectrum files from a batch of samples using a mass spectrometer. At 4002, an initial sample is selected. In various implementations, the orchestration logic may instruct the instrument logic 1004 to begin generating raw mass spectrum files for the batch of samples. For example, the instrument logic 1004 may direct an automated sample preparation platform and/or mass spectrometer to select the initial sample for processing. Examples of suitable automated sample preparation platforms include the AccelerOme Automated Sample Preparation platform available from Thermo Fisher Scientific Inc. At 4004, the instrument logic 1004 directs the automated sample preparation platform to prepare the selected sample. In some embodiments, the automated sample preparation platform may extract proteins from a biological sample. For example, the automated sample preparation platform may use protein denaturation, reduction, and/or alkylation to break down disulfide bonds and stabilize the proteins in the sample. In various implementations, the automated sample preparation platform may apply proteolysis techniques to the sample. For example, the automated sample preparation platform may apply enzymes - such as proteases - to the sample to digest proteins in the sample into smaller peptide fragments. In some examples, the automated sample preparation platform and/or mass spectrometer may separate peptides in the sample using liquid chromatography techniques - such as reverse-phase liquid chromatography.

At 4006, the instrument logic 1004 directs the mass spectrometer to ionize the prepared sample. In various implementations, the mass spectrometer may ionize the separated peptides in the prepared sample using techniques such as electrospray ionization or matrix-assisted laser desorption/ionization. At 4008, the instrument logic 1004 directs the mass spectrometer to perform ion separation on the ionized sample. In various implementations, the mass spectrometer may separate the ionized samples based on their mass-to-charge ratio (m/z). At 4010, the instrument logic 1004 directs the mass spectrometer to detect the separated ions. In various implementations, the mass spectrometer may perform tandem mass spectrometry. For example, the mass spectrometer may select specific precursor/peptide ions and fragment them using fragmentation techniques - such as collision-induced dissociation techniques. At 4012, the instrument logic 1004 directs the mass spectrometer to generate mass spectra from the detected separated ions. For example, the mass spectrometer may analyze the resulting ion fragments to generate a tandem mass spectra.

At 4014, the instrument logic 1004 determines whether another unprocessed sample exists in the batch of samples. In response to determining that there is another unprocessed sample in the batch ("YES" at decision block 4014), the instrument logic 1004 directs the automated sample preparation platform to select the next sample at 4016 and prepare the selected sample at 4004. In response to determining that there is not another unprocessed sample in the batch ("NO" at decision block 4014), the instrument logic 1004 saves the generated mass spectra for the processed samples as raw spectrum files for the batch of samples.

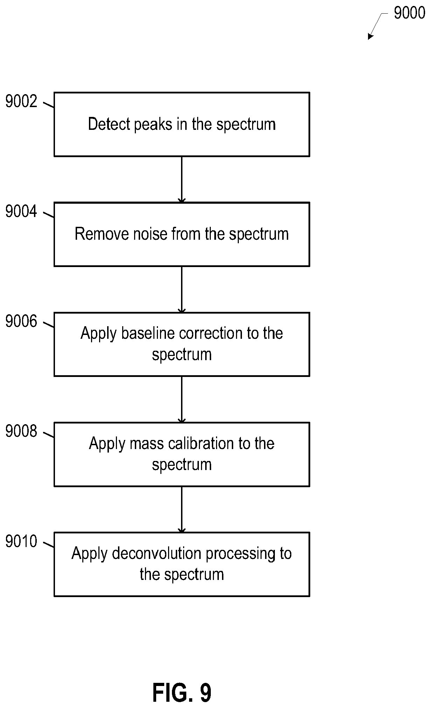

FIGS. 5A-5B is a flowchart of an example process 5000 for processing raw spectrum files to generate result files (such as updated or refined spectrum match files). At 5002, the analysis logic 1006 loads a raw spectrum file. At 5004, the analysis logic 1006 loads a screening list. At 5006, the analysis logic 1006 loads a protein database. In various implementations, the protein database includes reference protein sequences for organisms and/or samples of interest. At 5008, the analysis logic 1006 preprocesses the raw spectrum file. Additional details associated with preprocessing the raw spectrum file will be described further on in this specification with reference to FIG. 9. At 5010, the analysis logic 1006 generates a theoretical spectrum for each peptide sequence in the loaded protein database. In various implementations, the analysis logic 1006 calculates expected mass-to-charge ratios (m/z) for peptide fragments in the protein database. In some embodiments, the expected mass-to-charge ratios (m/z) may be calculated based on the fragmentation technique used by the mass spectrometer. At 5012, the analysis logic 1006 matches spectra in the preprocessed spectrum file loaded at 5002 with the theoretical spectra generated at 5010. In some examples, the analysis logic 1006 may also calculate a score for each match that reflects a closeness between the spectrum in the raw spectrum file and the matched theoretical spectrum. In some implementations, blocks 5006, 5010, and 5012 are not performed but rather, a database search engine may perform all or some of this function (e.g., in silico) for each spectrum file (and precursor mass). Also, in some implementations, the database search engine may perform this functionality differently from the functionality illustrated in and described with respect to FIG. 5A.

At 5014, the analysis logic 1006 determines whether the screening list was loaded at 5004. In response to determining that the screening list was not loaded ("NO" at decision block 5014), the analysis logic 1006 discards matched spectra having scores below a threshold at 5016. At 5018, the analysis logic 1006 saves the remaining matched spectra, associated peptides, and/or scores to the spectrum match file. In response to determining that the screening list was loaded ("YES" at decision block 5014), the analysis logic 1006 determines whether the screening list includes an inclusion list at 5020. In response to determining that the screening list includes the inclusion list ("YES" at decision block 5020), the analysis logic 1006 discards matched spectra (i) that are not on the inclusion list and (ii) that have scores below a threshold at 5022. The analysis logic 1006 determines whether the screening list includes an exclusion list at 5024. In response to determining that the screening list does not include the inclusion list ("NO" at decision block 5020), the analysis logic 1006 determines whether the screening list includes the exclusion list at 5024. In response to determining that the screening list includes the exclusion list ("YES" at decision block 5024), the analysis logic discards matched spectra that are on the exclusion list at 5026 and saves the remaining matched spectra, associated peptides, and/or scores to the result file at 5028.

FIG. 6 is a flowchart of an example process 6000 for generating a screening list from spectrum match files. At 6002, the analysis logic 1006 loads the spectrum files. At 6004, the analysis logic 1006 processes the loaded spectrum files and identifies peptides corresponding to the spectra of the loaded spectrum files. For example, the analysis logic 1006 may identify peptides as previously described with reference to 5010 and 5012. At 6006, the analysis logic 1006 determines a frequency of each identified peptide's appearance across all identified peptides in the batch of loaded spectrum files. At 6008, the analysis logic 1006 selects an initial identified peptide. At 6010, the analysis logic 1006 determines whether the identified peptide's frequency of appearance is above a threshold. In various implementations, the threshold may be a minimum frequency at which a peptide must appear across the batch of match spectra to be considered common. In response to determining that the selected identified peptide's frequency of appearance is greater than or equal to the threshold ("YES" at decision block 6010), the analysis logic 1006 adds the selected identified peptide to an inclusion list at 6012 and determines whether another identified peptide that has not yet been processed at 6010 is present in the batch at 6014. In response to determining that the frequency of appearance of the selected identified peptide is not greater than or equal to the threshold ("NO" at decision block 6010), the analysis logic 1006 determines whether another identified peptide that has not yet been processed at 6010 is present in the batch at 6014.

At 6014, the analysis logic 1006 determines whether another identified peptide that has not yet been processed at 6010 is present in the batch at 6014. In response to determining that another unprocessed identified peptide is present ("YES" at decision block 6014), the analysis logic 1006 selects the next identified peptide at 6016 and determines whether the frequency of appearance for that selected identified peptide is greater than or equal to the threshold at 6010. In response to determining that another unprocessed identified peptide is not present ("NO" at decision block 6014), the analysis logic 1006 saves the inclusion list at 6018.

FIG. 7 is a flowchart of an example process 7000 for generating a screening list from spectrum match files. At 7002, the analysis logic 1006 loads the spectrum match files. At 7004, the analysis logic 1006 sets a minimum intensity spectrum and filters out low-intensity peaks - such as peaks below the minimum intensity threshold - from the loaded spectrum files and saves the filtered loaded spectrum files as filtered spectrum files. At 7006, the analysis logic 1006 processes the filtered spectrum files to identify peptides. For example, the analysis logic 1006 may identify peptides as previously described with reference to 5010 and 5012. At 7008, the analysis logic 1006 counts a number of occurrences of each identified peptide in the filtered spectrum files. At 7010, the analysis logic 1006 sets a minimum occurrence threshold for peptides to be considered a contaminant. At 7012, the analysis logic 1006 selects an initial identified peptide from the filtered spectrum files.

At 7014, the analysis logic 1006 determines whether the number of occurrences of the selected identified peptide is below the minimum occurrence threshold. In response to determining that the number of occurrences of the selected identified peptide is below the minimum occurrence threshold ("YES" at decision block 7014), the analysis logic 1006 adds the selected identified peptide to an exclusion list at 7016 and determines whether another identified peptide that has not yet been processed at 7014 is present in the batch at 7018. In response to determining that the number of occurrences of the selected identified peptide is not below the minimum occurrence threshold ("NO" at decision block 7014), the analysis logic determines whether another unprocessed identified peptide is present at 7018. In response to determining that another unprocessed peptide is present in the batch of filtered spectrum files ("YES" at 7018), the analysis logic 1006 selects the next identified peptide from the filtered spectrum files and determines whether the number of occurrences of the selected identified peptide is above the minimum occurrence threshold at 7014. In response to determining that another unprocessed peptide is not present in the batch of filtered spectrum files ("NO" at 7018), the analysis logic 1006 saves the exclusion list at 7022.

FIG. 8 is a flowchart of an example process 8000 for generating a results list from spectrum match files. At 8002, the analysis logic 1006 combines spectrum match files into a single data structure. In various implementations, the single data structure may be a list or table, or a relational database. At 8004, the analysis logic 1006 filters the spectrum match files based on quality criteria. In some embodiments, the quality criteria may include false discovery rate, precursor mass tolerance, and/or search engine thresholds. Filtering the spectrum match files improves the overall confidence of the spectrum match files by removing low confidence identifications. At 8006 the analysis logic 1006 groups the spectrum match files into peptide and/or protein identifications. In various implementations, the analysis logic 1006 groups the filtered spectrum match files into peptide and protein identifications by aggregating spectrum match files with the same peptide sequences and/or considering shared or unique peptides among different proteins. At 8008, the analysis logic 1006 calculates peptide and/or protein abundances based on the intensities and/or spectral counts of the spectrum match files. At 8010, the analysis logic 1006 performs statistical tests on the spectrum match files to identify significantly altered proteins between different experiments. At 8012, the analysis logic 1006 generates a results list containing information about peptide and/or protein identifications.

FIG. 9 is a flowchart of an example process 9000 for preprocessing a raw spectrum file. At 9002, the analysis logic 1006 detects peaks in the acquired mass spectrum. For example, the analysis logic 1006 may find local maxima in the mass spectrum using derivative analysis techniques, wavelet-based methods, or other techniques. At 9004, the analysis logic 1006 removes noise from the mass spectrum. Electronic noise, chemical noise, and/or random fluctuations in the signal may introduce noise into mass spectra. Noise removal improves the overall signal-to-noise-ratio and enhances the quality of the mass spectrum. In various implementations, the analysis logic 1006 may apply moving average filtering techniques, median filtering techniques, Savitzky-Golay filters, and/or wavelet-based denoising techniques. At 9006, the analysis logic 1006 applies baseline correction to the mass spectrum. The baseline of the mass spectrum represents the background signal. Correcting the baseline ensure that peak heights accurately represent ion intensities. Examples of suitable baseline correction methods include polynomial function fitting techniques, asymmetric least squares algorithms, or other techniques suitable for modeling the baseline and then subtracting the modeled baseline from the original mass spectrum data. At 9008, the analysis logic 1006 applies mass calibration to the mass spectrum. Factors such as instrument drift and/or variations in the mass-to-charge ratios can affect the mass accuracy of acquired spectra. Mass calibration adjusts the mass-to-charge ratios to correct for these inaccuracies. In various implementations, the analysis logic 1006 may perform mass calibration by using calibration data - for example, calibration data representative of known reference compounds. In some examples, the analysis logic 1006 may apply a mass correction function that accounts for observed mass deviations.

At 9010, the analysis logic 1006 applies deconvolution processing to the mass spectrum. Mass spectrum data is typically represented as a series of peaks, with each peak indicating an intensity of a specific mass-to-charge ratio. However, peaks may overlap when multiple ions with similar mass-to-charge ratios co-elute. This can make it differentiate between these multiple ions in the mass spectrum. Applying deconvolution algorithms to the mass spectrum data (i) resolves overlapping peaks, allowing for accurate peak assignment and identification, (ii) separates co-eluting or overlapping isotopic peaks, improving a database search's accuracy, and/or (iii) simplifies the mass spectrum by reducing the number of peaks, improving the efficiency and accuracy of database searches. Suitable deconvolution techniques include maximum-entropy-based methods, peak fitting approaches, and mathematical transformations. Examples of peak fitting approaches include methods involving fitting a series of predefined peak shapes - such as Gaussian or Lorentzian functions - to the mass spectrum in order to find the combination of peak shapes and positions that best represent the observed data. Examples of suitable mathematical transformations include mathematical transformations that separate overlapping peaks, such as Fourier transformations, wavelet transformations, and/or the Savitzky-Golay method.

The scientific instrument support methods disclosed herein may include interactions with a human user (e.g., via the user local computing device 12020 discussed herein with reference to FIG. 12). These interactions may include providing information to the user (e.g., information regarding the operation of a scientific instrument such as the scientific instrument 12010 of FIG. 12, information regarding a sample being analyzed or other test or measurement performed by a scientific instrument, information retrieved from a local or remote database, or other information) or providing an option for a user to input commands (e.g., to control the operation of a scientific instrument such as the scientific instrument 10010 of FIG. 12, or to control the analysis of data generated by a scientific instrument), queries (e.g., to a local or remote database), or other information. In some embodiments, these interactions may be performed through a graphical user interface (GUI) that includes a visual display on a display device (e.g., the display device 11010 discussed herein with reference to FIG. 11) that provides outputs to the user and/or prompts the user to provide inputs (e.g., via one or more input devices, such as a keyboard, mouse, trackpad, or touchscreen, included in the other I/O devices 11012 discussed herein with reference to FIG. 11). The scientific instrument support systems disclosed herein may include any suitable GUIs for interaction with a user.

FIG. 10 depicts an example GUI 10000 that may be used in the performance of some or all of the support methods disclosed herein, in accordance with various embodiments. As noted above, the GUI 10000 may be provided on a display device (e.g., the display device 11010 discussed herein with reference to FIG. 11) of a computing device (e.g., the computing device 11000 discussed herein with reference to FIG. 11) of a scientific instrument support system (e.g., the scientific instrument support system 12000 discussed herein with reference to FIG. 12), and a user may interact with the GUI 10000 using any suitable input device (e.g., any of the input devices included in the other I/O devices 11012 discussed herein with reference to FIG. 11) and input technique (e.g., movement of a cursor, motion capture, facial recognition, gesture detection, voice recognition, actuation of buttons, etc.).

The GUI 10000 may include a data display region 10002, a data analysis region 10004, a scientific instrument control region 10006, and a settings region 10008. The particular number and arrangement of regions depicted in FIG. 10 is simply illustrative, and any number and arrangement of regions, including any desired features, may be included in a GUI 10000. In some examples, the GUI 10000 may implement user interfaces previously described with reference to FIGS. 2A-3C.

The data display region 10002 may display data generated by a scientific instrument (e.g., the scientific instrument 12010 discussed herein with reference to FIG. 12). For example, the data display region 10002 may display graphical representations of mass spectra - such as mass spectra discussed with reference to FIGS. 2A-9. In various implementations, the data display region 10002 may implement the data display region previously described with reference to FIGS. 2A-3C.

The data analysis region 10004 may display the results of data analysis (e.g., the results of analyzing the data illustrated in the data display region 10002 and/or other data). For example, the data analysis region 10004 may display results lists discussed with reference to FIGS. 2A-3C and 8. In some embodiments, the data display region 10002 and the data analysis region 10004 may be combined in the GUI 10000 (e.g., to include data output from a scientific instrument, and some analysis of the data, in a common graph or region). In various implementations, the data analysis region 10004 may implement the data analysis region previously described with reference to FIGS. 2A-3C.

The scientific instrument control region 10006 may include options that allow the user to control a scientific instrument (e.g., the scientific instrument 12010 discussed herein with reference to FIG. 12). For example, the scientific instrument control region 10006 may include selectable elements allowing the user to command scientific instruments such as the previously described mass spectrometers and/or automated sample preparation platforms - for example, at any of the steps of FIGS. 2A-9. In various implementations, the scientific instrument control region 10006 may implement the control region previously described with reference to FIGS. 2A-3C. The settings region 10008 may include options that allow the user to control the features and functions of the GUI 10000 (and/or other GUIs) and/or perform common computing operations with respect to the data display region 10002 and data analysis region 10004 (e.g., saving data on a storage device, such as the storage device 11004 discussed herein with reference to FIG. 11, sending data to another user, labeling data, etc.

As noted above, the scientific instrument support module 1000 may be implemented by one or more computing devices. FIG. 11 is a block diagram of a computing device 11000 that may perform some or all of the scientific instrument support methods disclosed herein, in accordance with various embodiments. In some embodiments, the scientific instrument support module 1000 may be implemented by a single computing device 11000 or by multiple computing devices 11000. Further, as discussed below, a computing device 11000 (or multiple computing devices 11000) that implements the scientific instrument support module 1000 may be part of one or more of the scientific instrument 12010, the user local computing device 12020, the service local computing device 12030, or the remote computing device 12040 of FIG. 12.

The computing device 11000 of FIG. 11 is illustrated as having a number of components, but any one or more of these components may be omitted or duplicated, as suitable for the application and setting. In some embodiments, some or all of the components included in the computing device 11000 may be attached to one or more motherboards and enclosed in a housing (e.g., including plastic, metal, and/or other materials). In some embodiments, some these components may be fabricated onto a single system-on-a-chip (SoC) (e.g., an SoC may include one or more processing devices 11002 and one or more storage devices 11004). Additionally, in various embodiments, the computing device 11000 may not include one or more of the components illustrated in FIG. 11, but may include interface circuitry (not shown) for coupling to the one or more components using any suitable interface (e.g., a Universal Serial Bus (USB) interface, a High-Definition Multimedia interface (HDMI) interface, a Controller Area Network (CAN) interface, a Serial Peripheral interface (SPI) interface, an Ethernet interface, a wireless interface, or any other appropriate interface). For example, the computing device 11000 may not include a display device 11010, but may include display device interface circuitry (e.g., a connector and driver circuitry) to which a display device 11010 may be coupled.

The computing device 11000 may include a processing device 11002 (e.g., one or more processing devices). As used herein, the term "processing device" may refer to any device or portion of a device that processes electronic data from registers and/or memory to transform that electronic data into other electronic data that may be stored in registers and/or memory. The processing device 11002 may include one or more digital signal processors (DSPs), application-specific integrated circuits (ASICs), central processing units (CPUs), graphics processing units (GPUs), cryptoprocessors (specialized processors that execute cryptographic algorithms within hardware), server processors, or any other suitable processing devices.

The computing device 11000 may include a storage device 11004 (e.g., one or more storage devices). The storage device 11004 may include one or more memory devices such as random access memory (RAM) (e.g., static RAM (SRAM) devices, magnetic RAM (MRAM) devices, dynamic RAM (DRAM) devices, resistive RAM (RRAM) devices, or conductive-bridging RAM (CBRAM) devices), hard drive-based memory devices, solid-state memory devices, networked drives, cloud drives, or any combination of memory devices. In some embodiments, the storage device 11004 may include memory that shares a die with a processing device 11002. In such an embodiment, the memory may be used as cache memory and may include embedded dynamic random access memory (eDRAM) or spin transfer torque magnetic random access memory (STT-MRAM), for example. In some embodiments, the storage device 11004 may include non-transitory computer readable media having instructions thereon that, when executed by one or more processing devices (e.g., the processing device 11002), cause the computing device 11000 to perform any appropriate ones of or portions of the methods disclosed herein.

The computing device 11000 may include an interface device 11006 (e.g., one or more interface devices 4006). The interface device 11006 may include one or more communication chips, connectors, and/or other hardware and software to govern communications between the computing device 11000 and other computing devices. For example, the interface device 11006 may include circuitry for managing wireless communications for the transfer of data to and from the computing device 11000. The term "wireless" and its derivatives may be used to describe circuits, devices, systems, methods, techniques, communications channels, etc., that may communicate data through the use of modulated electromagnetic radiation through a nonsolid medium. The term does not imply that the associated devices do not contain any wires, although in some embodiments they might not. Circuitry included in the interface device 11006 for managing wireless communications may implement any of a number of wireless standards or protocols, including but not limited to Institute for Electrical and Electronic Engineers (IEEE) standards including Wi-Fi (IEEE 802.11 family), IEEE 802.16 standards (e.g., IEEE 802.16-2005 Amendment), Long-Term Evolution (LTE) project along with any amendments, updates, and/or revisions (e.g., advanced LTE project, ultra mobile broadband (UMB) project (also referred to as "3GPP2"), etc.). In some embodiments, circuitry included in the interface device 11006 for managing wireless communications may operate in accordance with a Global System for Mobile Communication (GSM), General Packet Radio Service (GPRS), Universal Mobile Telecommunications System (UMTS), High Speed Packet Access (HSPA), Evolved HSPA (E-HSPA), or LTE network. In some embodiments, circuitry included in the interface device 11006 for managing wireless communications may operate in accordance with Enhanced Data for GSM Evolution (EDGE), GSM EDGE Radio Access Network (GERAN), Universal Terrestrial Radio Access Network (UTRAN), or Evolved UTRAN (E-UTRAN). In some embodiments, circuitry included in the interface device 11006 for managing wireless communications may operate in accordance with Code Division Multiple Access (CDMA), Time Division Multiple Access (TDMA), Digital Enhanced Cordless Telecommunications (DECT), Evolution-Data Optimized (EV-DO), and derivatives thereof, as well as any other wireless protocols that are designated as 3G, 4G, 5G, and beyond. In some embodiments, the interface device 11006 may include one or more antennas (e.g., one or more antenna arrays) to receipt and/or transmission of wireless communications.

In some embodiments, the interface device 11006 may include circuitry for managing wired communications, such as electrical, optical, or any other suitable communication protocols. For example, the interface device 11006 may include circuitry to support communications in accordance with Ethernet technologies. In some embodiments, the interface device 11006 may support both wireless and wired communication, and/or may support multiple wired communication protocols and/or multiple wireless communication protocols. For example, a first set of circuitry of the interface device 11006 may be dedicated to shorter-range wireless communications such as Wi-Fi or Bluetooth, and a second set of circuitry of the interface device 11006 may be dedicated to longer-range wireless communications such as global positioning system (GPS), EDGE, GPRS, CDMA, WiMAX, LTE, EV-DO, or others. In some embodiments, a first set of circuitry of the interface device 11006 may be dedicated to wireless communications, and a second set of circuitry of the interface device 11006 may be dedicated to wired communications.

The computing device 11000 may include battery/power circuitry 11008. The battery/power circuitry 11008 may include one or more energy storage devices (e.g., batteries or capacitors) and/or circuitry for coupling components of the computing device 11000 to an energy source separate from the computing device 11000 (e.g., AC line power).

The computing device 11000 may include a display device 11010 (e.g., multiple display devices). The display device 11010 may include any visual indicators, such as a heads-up display, a computer monitor, a projector, a touchscreen display, a liquid crystal display (LCD), a light-emitting diode display, or a flat panel display.

The computing device 11000 may include other input/output (I/O) devices 11012. The other I/O devices 11012 may include one or more audio output devices (e.g., speakers, headsets, earbuds, alarms, etc.), one or more audio input devices (e.g., microphones or microphone arrays), location devices (e.g., GPS devices in communication with a satellite-based system to receive a location of the computing device 11000, as known in the art), audio codecs, video codecs, printers, sensors (e.g., thermocouples or other temperature sensors, humidity sensors, pressure sensors, vibration sensors, accelerometers, gyroscopes, etc.), image capture devices such as cameras, keyboards, cursor control devices such as a mouse, a stylus, a trackball, or a touchpad, bar code readers, Quick Response (QR) code readers, or radio frequency identification (RFID) readers, for example.

The computing device 11000 may have any suitable form factor for its application and setting, such as a handheld or mobile computing device (e.g., a cell phone, a smart phone, a mobile internet device, a tablet computer, a laptop computer, a netbook computer, an ultrabook computer, a personal digital assistant (PDA), an ultra mobile personal computer, etc.), a desktop computing device, or a server computing device or other networked computing component.

One or more computing devices implementing any of the scientific instrument support modules or methods disclosed herein may be part of a scientific instrument support system. FIG. 12 is a block diagram of an example scientific instrument support system 12000 in which some or all of the scientific instrument support methods disclosed herein may be performed, in accordance with various embodiments. The scientific instrument support modules and methods disclosed herein (e.g., the scientific instrument support module 1000 of FIG. 1 and the processes 2000-9000 of FIGS. 2A-9) may be implemented by one or more of the scientific instrument 12010, the user local computing device 12020, the service local computing device 12030, or the remote computing device 12040 of the scientific instrument support system 12000.

Any of the scientific instrument 12010, the user local computing device 12020, the service local computing device 12030, or the remote computing device 12040 may include any of the embodiments of the computing device 11000 discussed herein with reference to FIG. 11, and any of the scientific instrument 12010, the user local computing device 12020, the service local computing device 12030, or the remote computing device 12040 may take the form of any appropriate ones of the embodiments of the computing device 11000 discussed herein with reference to FIG. 11.

The scientific instrument 12010, the user local computing device 12020, the service local computing device 12030, or the remote computing device 12040 may each include a processing device 12002, a storage device 12004, and an interface device 12006. The processing device 12002 may take any suitable form, including the form of any of the processing devices 11002 discussed herein with reference to FIG. 11, and the processing devices 12002 included in different ones of the scientific instrument 12010, the user local computing device 12020, the service local computing device 12030, or the remote computing device 12040 may take the same form or different forms. The storage device 12004 may take any suitable form, including the form of any of the storage devices 11004 discussed herein with reference to FIG. 11, and the storage devices 12004 included in different ones of the scientific instrument 12010, the user local computing device 12020, the service local computing device 12030, or the remote computing device 12040 may take the same form or different forms. The interface device 12006 may take any suitable form, including the form of any of the interface devices 11006 discussed herein with reference to FIG. 11, and the interface devices 12006 included in different ones of the scientific instrument 12010, the user local computing device 12020, the service local computing device 12030, or the remote computing device 12040 may take the same form or different forms.

The scientific instrument 12010, the user local computing device 12020, the service local computing device 12030, and the remote computing device 12040 may be in communication with other elements of the scientific instrument support system 12000 via communication pathways 12008. The communication pathways 12008 may communicatively couple the interface devices 12006 of different ones of the elements of the scientific instrument support system 12000, as shown, and may be wired or wireless communication pathways (e.g., in accordance with any of the communication techniques discussed herein with reference to the interface devices 11006 of the computing device 11000 of FIG. 11). The particular scientific instrument support system 12000 depicted in FIG. 12 includes communication pathways between each pair of the scientific instrument 12010, the user local computing device 12020, the service local computing device 12030, and the remote computing device 12040, but this "fully connected" implementation is simply illustrative, and in various embodiments, various ones of the communication pathways 12008 may be absent. For example, in some embodiments, a service local computing device 12030 may not have a direct communication pathway 12008 between its interface device 12006 and the interface device 12006 of the scientific instrument 12010, but may instead communicate with the scientific instrument 12010 via the communication pathway 12008 between the service local computing device 12030 and the user local computing device 12020 and the communication pathway 12008 between the user local computing device 12020 and the scientific instrument 12010.

The scientific instrument 12010 may include any appropriate scientific instrument, such as a mass spectrometer or an automated sample preparation platform. In various implementations, the scientific instrument 12010 may include multiple scientific instruments - such as one or more mass spectrometers and one or more automated sample preparation platforms. Examples of suitable automated sample preparation platforms include any of those previously discussed with reference to FIGS. 2A-9. Examples of suitable mass spectrometers may include quadrupole mass spectrometers, time-of-flight mass spectrometers, ion trap mass spectrometers, triple quadrupole mass spectrometer, quadrupole time-of-flight mass spectrometers, and/or Fourier-transform ion cyclotron resonance mass spectrometers. In various implementations, suitable mass spectrometers may include any of the mass spectrometers available from Thermo Fisher Scientific Inc. - such as the Orbitrap and Q Exactive^{™} lines of mass spectrometers.

The user local computing device 12020 may be a computing device (e.g., in accordance with any of the embodiments of the computing device 11000 discussed herein) that is local to a user of the scientific instrument 12010. In some embodiments, the user local computing device 12020 may also be local to the scientific instrument 12010, but this need not be the case; for example, a user local computing device 12020 that is in a user's home or office may be remote from, but in communication with, the scientific instrument 12010 so that the user may use the user local computing device 12020 to control and/or access data from the scientific instrument 12010. In some embodiments, the user local computing device 12020 may be a laptop, smartphone, or tablet device. In some embodiments the user local computing device 12020 may be a portable computing device.

The service local computing device 12030 may be a computing device (e.g., in accordance with any of the embodiments of the computing device 11000 discussed herein) that is local to an entity that services the scientific instrument 12010. For example, the service local computing device 12030 may be local to a manufacturer of the scientific instrument 12010 or to a third-party service company. In some embodiments, the service local computing device 12030 may communicate with the scientific instrument 12010, the user local computing device 12020, and/or the remote computing device 12040 (e.g., via a direct communication pathway 12008 or via multiple "indirect" communication pathways 12008, as discussed above) to receive data regarding the operation of the scientific instrument 12010, the user local computing device 12020, and/or the remote computing device 12040 (e.g., the results of self-tests of the scientific instrument 12010, calibration coefficients used by the scientific instrument 12010, the measurements of sensors associated with the scientific instrument 12010, etc.). In some embodiments, the service local computing device 12030 may communicate with the scientific instrument 12010, the user local computing device 12020, and/or the remote computing device 12040 (e.g., via a direct communication pathway 12008 or via multiple "indirect" communication pathways 12008, as discussed above) to transmit data to the scientific instrument 12010, the user local computing device 12020, and/or the remote computing device 12040 (e.g., to update programmed instructions, such as firmware, in the scientific instrument 12010, to initiate the performance of test or calibration sequences in the scientific instrument 12010, to update programmed instructions, such as software, in the user local computing device 12020 or the remote computing device 12040, etc.). A user of the scientific instrument 12010 may utilize the scientific instrument 12010 or the user local computing device 12020 to communicate with the service local computing device 12030 to report a problem with the scientific instrument 12010 or the user local computing device 12020, to request a visit from a technician to improve the operation of the scientific instrument 12010, to order consumables or replacement parts associated with the scientific instrument 12010, or for other purposes.

The remote computing device 12040 may be a computing device (e.g., in accordance with any of the embodiments of the computing device 11000 discussed herein) that is remote from the scientific instrument 12010 and/or from the user local computing device 12020. In some embodiments, the remote computing device 12040 may be included in a datacenter or other large-scale server environment. In some embodiments, the remote computing device 12040 may include network-attached storage (e.g., as part of the storage device 12004). The remote computing device 12040 may store data generated by the scientific instrument 12010, perform analyses of the data generated by the scientific instrument 12010 (e.g., in accordance with programmed instructions), facilitate communication between the user local computing device 12020 and the scientific instrument 12010, and/or facilitate communication between the service local computing device 12030 and the scientific instrument 12010.

In some embodiments, one or more of the elements of the scientific instrument support system 12000 illustrated in FIG. 12 may not be present. Further, in some embodiments, multiple ones of various ones of the elements of the scientific instrument support system 12000 of FIG. 12 may be present. For example, a scientific instrument support system 12000 may include multiple user local computing devices 12020 (e.g., different user local computing devices 12020 associated with different users or in different locations). In another example, a scientific instrument support system 12000 may include multiple scientific instruments 12010, all in communication with service local computing device 12030 and/or a remote computing device 12040; in such an embodiment, the service local computing device 12030 may monitor these multiple scientific instruments 12010, and the service local computing device 12030 may cause updates or other information may be "broadcast" to multiple scientific instruments 12010 at the same time. Different ones of the scientific instruments 12010 in a scientific instrument support system 12000 may be located close to one another (e.g., in the same room) or farther from one another (e.g., on different floors of a building, in different buildings, in different cities, etc.). In some embodiments, a scientific instrument 12010 may be connected to an Internet-of-Things (IoT) stack that allows for command and control of the scientific instrument 12010 through a web-based application, a virtual or augmented reality application, a mobile application, and/or a desktop application. Any of these applications may be accessed by a user operating the user local computing device 12020 in communication with the scientific instrument 12010 by the intervening remote computing device 12040. In some embodiments, a scientific instrument 12010 may be sold by the manufacturer along with one or more associated user local computing devices 12020 as part of a local scientific instrument computing unit 12012.

In some embodiments, different ones of the scientific instruments 12010 included in a scientific instrument support system 12000 may be different types of scientific instruments 12010; for example, one scientific instrument 12010 may be a mass spectrometer, while another scientific instrument 12010 may be an automated sample preparation platform. In some such embodiments, the remote computing device 12040 and/or the user local computing device 12020 may combine data from different types of scientific instruments 12010 included in a scientific instrument support system 12000.

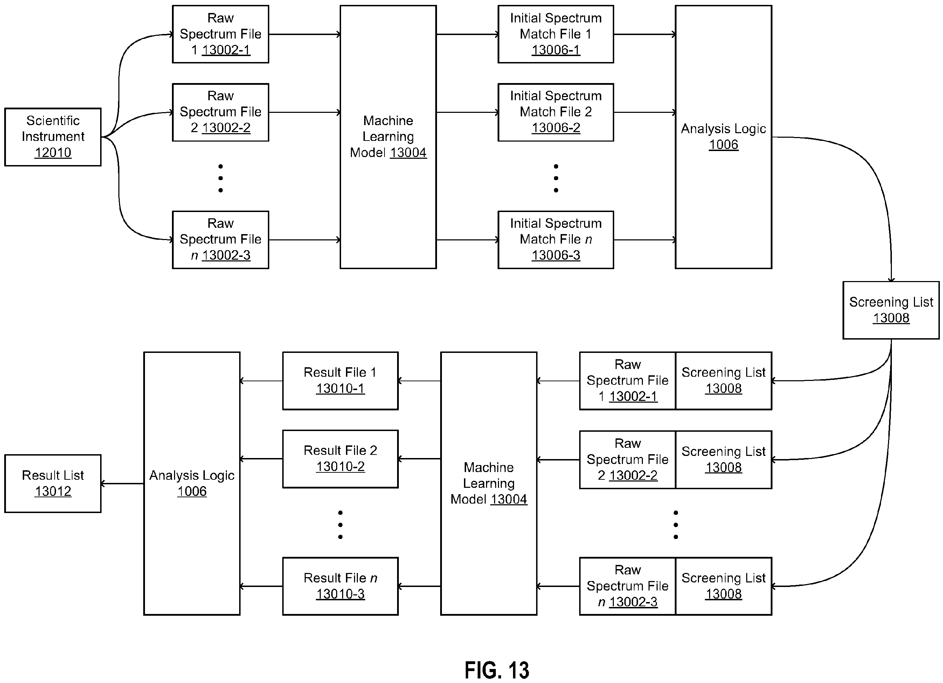

FIG. 13 is a block diagram of an example implementation of a portion of the first process 2000. As previously described with reference to process 2000, the analysis logic 1006 may load raw spectrum files for an entire batch of samples. As shown in the example of FIG. 13, the batch may include n samples. Thus, the analysis logic 1006 may load raw spectrum files 1-n. While only three raw spectrum files 13002-1, 13002-2, and 13002-3 are illustrated in FIG. 13, n may be any number - and so any number n of raw spectrum files may be loaded. The analysis logic 1006 loads each of the n raw spectrum files 13002-1-13002-3 to a machine learning model - such as machine learning model 13004. The machine learning model 13004 processes each of the n raw spectrum files 13002-1-13002-3 and generates an initial spectrum match file for each of the n raw spectrum files 13002-1-13002-3. Thus, as shown in the example of FIG. 13, the machine learning model 13004 may generate n initial spectrum match files 13006-1-13006-3 with each initial spectrum match file corresponding to a raw spectrum file. While only three initial spectrum match files 13006-1-13006-3 are shown in FIG. 13, n may be any number - and so any number n of initial spectrum match files may be generated. The analysis logic 1006 then parses the entire batch of n initial spectrum match files 13006-1-13006-3 to generate a screening list 14006.

The analysis logic 1006 provides each of the entire batch of n raw spectrum files 13002-1-13002-3 with the screening list 13008 to the machine learning model 13004. After processing each of the n raw spectrum files 13002-1-13002-3 with the screening list 13008, the machine learning model 13004 generates a result file for each of the *n* raw spectrum files 13002-1-13002-3. While only three raw spectrum files 13002-1-13002-3 and three result files 13010-1-13010-3 are illustrated in FIG. 13, n may be any number- and so any number of n raw spectrum match files may be provided to machine learning model 13004 to generate any number n of result files. Thus, as shown in the example of FIG. 13, the machine learning model 13004 may generate n result files 13010-1-13010-3 (with each result file corresponding to a raw spectrum file). The analysis logic 1006 then parses the entire batch of n result files 13010-1-13010-3 to generate a results list 13012. As shown in FIG. 13, some processes require (i) the entire batch of n result files 13002-1-13002-3 to be processed by machine learning model 13004 to generate a batch n initial spectrum match files 13006-1-13006-3 and (ii) the entire batch of n initial spectrum match files 13006-1-13006-3 to be processed by the analysis logic 1006 before the screening list 13008 can be generated.

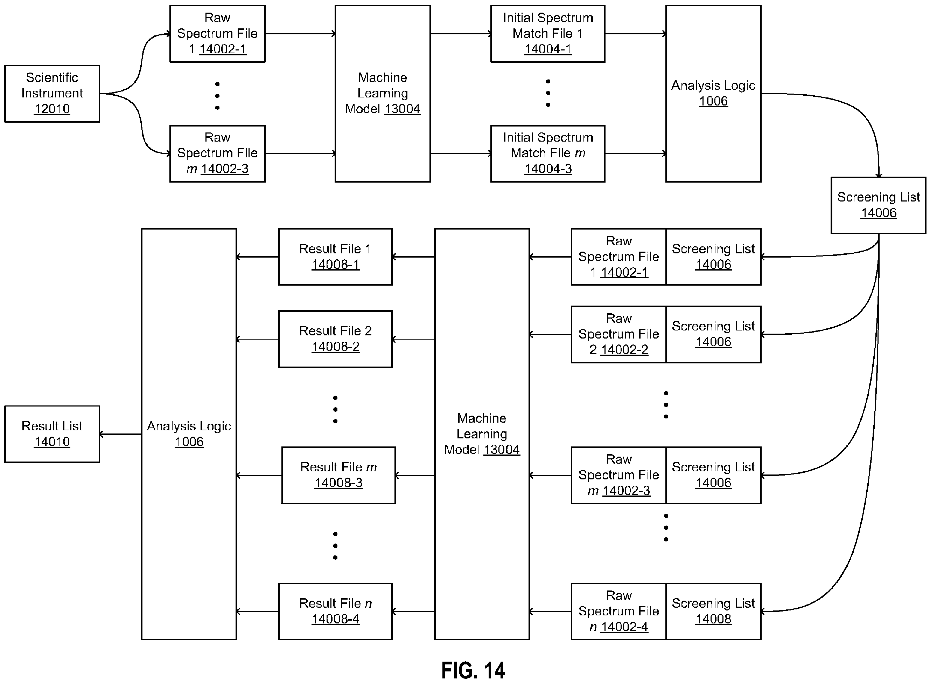

FIG. 14 is a block diagram of an example implementation of a portion of the second process 3000. As previously described with reference to process 3000, analysis logic 1006 may load raw spectrum files for an entire batch of *n* samples. Analysis logic 1006 may divide the batch of *n* raw spectrum files into a smaller subset of *m* raw spectrum files. As previously discussed, the size of m may be substantially smaller than the size of *n*. For example, in some embodiments *m* may be about 10% of *n.* In various implementations *m* may be about 20% of *n*. According to some examples, *m* may be about 25% of *n.* In other examples, *m* may be about 33% of *n.* In various embodiments, *m* may be about 50% of *n.* In some implementations, *m* may be about 67% of *n*. The analysis logic 1006 provides the subset of *m* raw spectrum files - illustrated in FIG. 14 as raw spectrum files 14002-1-14002-3 - to the machine learning model 13004 to generate *m* initial spectrum match files 14004-1-14004-3. While only two raw spectrum files 14002-1-14002-3 and two initial spectrum match files 14004-1-14004-3 are illustrated in FIG. 14, *m* may be any number - and so any number *m* of raw spectrum files may be loaded and any number *m* of initial spectrum match files may be generated. As shown in FIG. 14, the machine learning model 13004 may generate a corresponding initial spectrum match file for each of the subset of *m* raw spectrum files 14002-1-14002-3. The analysis logic 1006 then processes the subset of *m* initial spectrum match files 14004-1-14004-3 to generate a screening list 14006.

The analysis logic 1006 provides each of a second subset of the entire batch of *n* raw spectrum files 14002-1-14002-4 - or, as illustrated in the example of FIG. 14, the entire batch of *n* raw spectrum files 14002-1-14002-4 - to the machine learning model 13004 along with the screening list 14006 to generate a result file for each of the raw spectrum files input to the machine learning model 13004. While only four raw spectrum files 14002-1-14002-4 are illustrated in FIG. 14, n may be any number, and so the batch of *n* raw spectrum files may include any number of raw spectrum files. Similarly, while only four result files 14008-1-14008-4 are shown in FIG. 14, the machine learning model 1004 may generate any number *n* of result files based on the input raw spectrum files. The analysis logic 1006 then processes the result files 14008-1-14008-4 to generate a results list 14010.

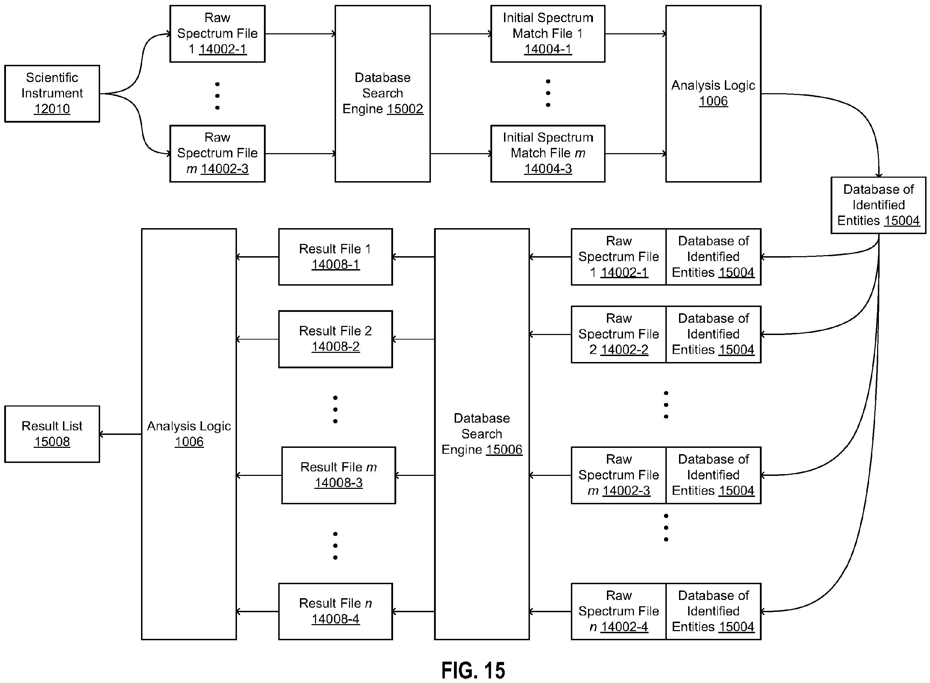

FIG. 15 is a block diagram of an example implementation of a portion of the second process 3000. As previously described with reference to process 3000, analysis logic 1006 loads raw spectrum files for an entire batch of *n* samples. The batch of *n* samples may include mass spectrometry data representing one or more samples. Analysis logic 1006 divides the batch of *n* raw spectrum files into a smaller subset of *m* raw spectrum files. Analysis logic 1006 provides the subset of *m* raw spectrum files - illustrated in FIG. 15 as raw spectrum files 14002-1-14002-3 to a database search engine 15002 to generate *m* initial spectrum match files 14004-1-14004-3. While only two raw spectrum files 14002-1-14002-3 and two initial spectrum match files 14004-1-14004-3 are shown in FIG. 14, *m* may be any number (and so any number *m* of raw spectrum files may be loaded and any number *m* of initial spectrum match files may be generated). As illustrated in FIG. 15, the database search engine 15002 may generate a corresponding initial spectrum match file for each of the subset of *m* raw spectrum files 14002-1-14002-3. The analysis logic 1006 then consolidates the results of the subset of *m* initial spectrum match files 14004-1-14004-3 to generate a database of identified entities file 15004.

In various implementations, analysis logic 1006 generates the database of identified entities 15004 by processing the initial mass spectrum files by comparing mass spectra from the initial mass spectrum files with entries from a reference database of measured spectra, post-processed spectra, and/or synthetic spectra. For example, at least one of a threshold, false detection rate, and/or spectral match score may be used to identify a minimum quality criterion for identified entities. Only entities above the minimum quality criterion are added to the database of identified entities 15004. In example embodiments, the reference database may include peptide spectra. In some examples, the reference database may include peptide sequences. In various implementations, the reference database may include synthetic spectra - which may be generated in real time or concurrently with the comparison process. In example implementations, the database of identified entities 15004 may include peptide sequences, peptide identifications, mass spectra of peptides, retention time and/or retention index information, and/or precursor ion information (such as masses, mass-to-charge ratios [m/z], and/or m/z windows).

In various implementations (not shown in FIG. 15), the database search engine 15002 reprocesses the batch of *m* raw spectrum files 14002-1-14002-3 using the database of identified entities 15004 to generate m result files (for example, to identify and/or quantitate entities based on the database of identified entities 15004) for the batch.

The analysis logic 1006 provides each of a second subset of the batch of n raw spectrum files 14002-1-14002-4 (or, as illustrated in FIG. 15, the entire batch of *n* raw spectrum files) along with the database of identified entities 15004 to a database search engine 15006 to generate a result file for each of the raw spectrum files input to the database search engine 15006. In various implementations, database search engine 15006 may be the same as database search engine 15002. In other examples, database search engine 15006 may be different than database search engine 15002. In various implementations, the database search engine 15006 generates the result files by comparing ion spectra or fragmentation spectra from the raw spectrum file with entries in the database of identified entities 15006. For example, the database search engine 15006 may specifically search the raw spectrum file for the entities in the database of identified entities 15004 (for example, using precursor information and/or retention time information).

In various implementations, the database search engine 15006 may process at least some of the second subset or the entire batch of n raw spectrum files to extend the database of identified entities 15004. For example, the database search engine 15006 re-searches the subset of *m* initial spectrum match files 14004-1-14004-3 and second subset or the entire batch of *n* result files to receive further identification and/or quantification information. This additional processing may be stopped when the growth rate of the database of identified entities 15004 falls below a threshold (for example, an average of less than 10, 1, 0.1, or 0.01 additional entries per spectrum file).

While only four raw spectrum files 14002-1-14002-4 are illustrated in FIG. 15, n may be any number, and so the batch of *n* raw spectrum files may include any number of raw spectrum files. Similarly, while only four result files 14008-1-14008-4 are shown in FIG. 15, the machine learning model 1004 may generate any number n of result files based on the input raw spectrum files. The analysis logic 1006 then processes the result files 14008-1-14008-4 to generate a results list 15008.

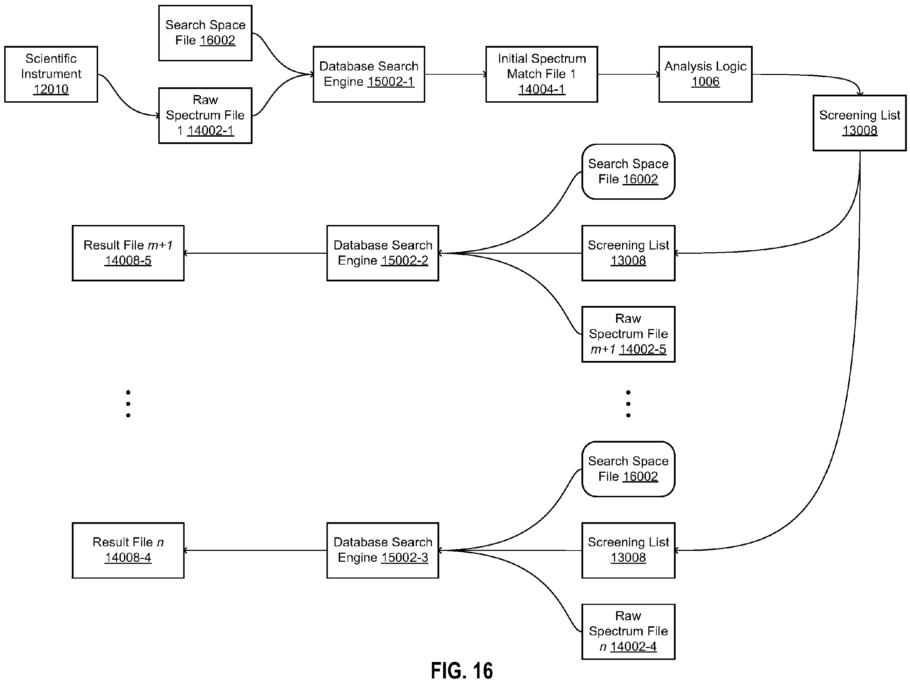

FIG. 16 is a block diagram of an example implementation of a portion of the second process 3000. As previously described with reference to process 3000, analysis logic 1006 loads a batch of raw spectrum files. For example, analysis logic 1006 creates a first set of raw spectrum files representing one or more samples. The first set of raw spectrum files may include raw spectrum file 14002-1. While only a single raw spectrum file from the first set of raw spectrum files is illustrated in FIG. 16, the first set of raw spectrum files may include any number of raw spectrum files. The analysis logic 1006 provides each raw spectrum file from the first set of raw spectrum files to a machine learning model - such as database search engine 15002-1 - along with a search space file 16002. The database search engine 15002-1 generates an initial spectrum match file - such as initial spectrum match file 14004-1 - for each raw spectrum file from the first set of raw spectrum files. The analysis logic 1006 consolidates and/or analyzes results of the first set of raw spectrum files - such as the initial spectrum match files - to generate a screening list 13008. In various implementations, the analysis logic generates screening list 13008 by merging high-confidence identifications from all searches into one screening list of identified entities (corresponding to a given experimental setup).

Analysis logic 1006 loads a second set of raw spectrum files, such as raw spectrum files 14002-5-14002-4. While only two raw spectrum files from the second set of raw spectrum files are shown in FIG. 16, the second set of raw spectrum files may include any number of raw spectrum files. Each raw spectrum file from the second set of raw spectrum files is provided - along with the screening list 13008 and, optionally, the search space file 16002 - to a database search engine. Each raw spectrum file from the second set of raw spectrum files may be provided to a same or different database search engine. For example, database search engine 15002-2 may be the same as or different from database search engine 15002-3. Similarly, database search engine 15002-1 may be the same as or different from database search engines 15002-2-15002-3. Each database search engine then generates a result file for a respective spectrum match file of the second set of raw spectrum files. While only two result files 14008-4-14004-5 are shown, the second set of raw spectrum files may be any size, and so any number of result files may be generated for the second set of raw spectrum files.

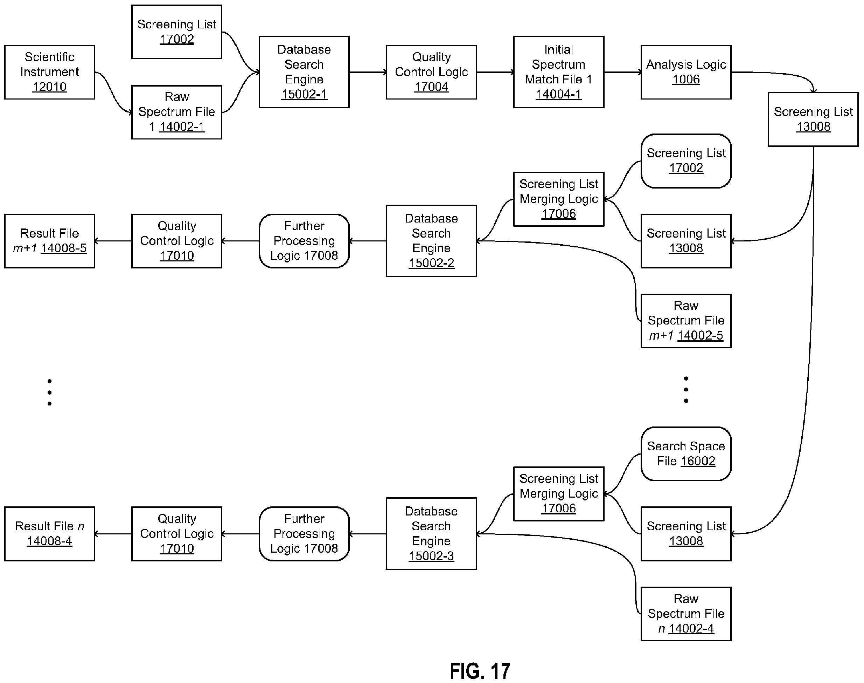

FIG. 17 is a block diagram of an example implementation of a portion of the second process 3000. As previously described with reference to process 3000, analysis logic 1006 loads a set of related mass spectrometry data from one or more connected studies - such as a batch of raw spectrum files. The mass spectrometry data may include mass, intensity, retention time, ion mobility property, physico-chemical property, and/or a location on a spatially arrange sample (such as a tissue, cell, or gel). The elements of the set of mass spectrometry data may include or represent individual samples from one or more studies, and may be related by one or more of: (i) a similarity of sample type (such as blood samples, cell culture samples, and/or tissue samples) and (ii) a similarity of data acquisition methods (such as being generated from liquid chromatography mass spectrometry [LC-MS], being generated from matrix-assisted laser desorption/ionization [MALDI] mass spectrometry, being generated the same liquid chromatography [LC] column, being prepared with the same separation agent, sharing a common sample preparation, sharing a same matrix, sharing a same mass spectrometry scheme [e.g., sharing a data-independent acquisition scheme with similar fragmentation windows and/or energies, sharing similar mass spectrometry settings, being generated by a same method such as stable isotope labeling by ammino acids in cell culture (SILAC), sharing isotopic mass tags, etc.]).

As shown in FIG. 17, analysis logic 1006 may create a first subset of the mass spectrometry data - such as a first set of raw spectrum files. The first set of raw spectrum files may include raw spectrum file 14002-1. While only a single raw spectrum file from the first set of raw spectrum files is illustrated in FIG. 17, the first set of raw spectrum files may include any number of raw spectrum files (including only a single raw spectrum file). Analysis logic 1006 provides each raw spectrum file from the first set of raw spectrum files and a screening list 17002 to a machine learning model - such as database search engine 15002-1. In various implementations, screening list 17002 may include a FASTA file and define a search space for database search engine 15002-1 (or any of the machine learning models used in process 3000). In some examples, database search engine 15002-1 processes the first set of raw spectrum files and generates an initial spectrum match file - such as initial spectrum match file 14004-1 - for each raw spectrum file from the first set of ra spectrum files. Analysis logic 1006 consolidates and/or analyzes results of the first set of raw spectrum files - such as the initial spectrum match files - to generate a screening list 13008.

In some embodiments, screening list 13008 may include a database of identified entities, and the entities may include peptides and/or proteins. In various implementations, database search engine 15002-1 may include quality control logic 17004, and entities in the database of identified entities may be selected in response to passing a quality control test. In some examples, the quality control test includes at least one of a false discovery rate test, meeting a minimum threshold (e.g., a minimum intensity or other spectral quality), meeting a minimum matching score (e.g., sharing a minimum number of peaks with a reference spectrum), and having a minimum number of occurrences within the subset. In some examples, quality control logic 17004 may be implemented as a machine learning model, such as the Percolator and/or mokapot semi-supervised learning techniques for peptide detection. In various implementations, entities in the database of identified entities may be represented by one or more of an entity identifier (e.g., a CAS Registry Number and/or a Swiss-Prot ID), a protein or peptide sequence, one or more masses from an MS or MS/MS spectrum (with or without intensity values), and one or more further physico-chemical properties (e.g., retention times and/or ion mobilities).

Analysis logic 1006 loads a second subset of the set of related mass spectrometry data - such as a second set of the batch of raw spectrum files. For example, analysis logic 1006 loads raw spectrum files 14005-2-14002-4. While only two raw spectrum files from the second set of raw spectrum files are shown in FIG. 16, the second set of raw spectrum files may include any number of raw spectrum files (including only one). Each raw spectrum file from the second set of raw spectrum files - along with screening list 13008 - to a database search engine. In some examples, screening list merging logic 17006 merges screening list 17002 with screening list 13008 and provides the merged screening list to the database search engine. Each raw spectrum file from the second set of raw spectrum files may be provided to a same or different database search engine. For example, database search engine 15002-2 may be the same as or different from database search engine 15002-3. Similarly, database search engine 15002-1 may be the same as or different from database search engines 15002-2-15002-3. Each database search engine then processes a respective spectrum match file of the second set of raw spectrum files along with screening list 13008 or the merged screening list to generate a result file.

In various implementations, the database search engine may exclude entities not present in the screening list 13008 or merged screening list from further processing. In some examples, the database search engine may include any entities contained in the screening list 13008 or merged screening list for further processing. In some embodiments, the database search engine may use screening list 17002 to identify further entities for addition to the second screening list. Already processed data may be retroactively reprocessed to include processing and further processing for new elements of screening list 13008. In various implementations, the database search engine may process the raw spectrum file by comparing mass spectrometry data (such as one or more of mass, intensity, retention time, and ion mobility) with selected reference library spectra. In some examples, the database search engine may process the raw spectrum file by comparing mass spectrometry data with synthetic spectra generated based on entities in the screening list 13008 or merged screening list. In some embodiments, the database search engine may identify entities present in the raw spectrum file by matching spectra from the raw spectrum file with reference library spectra and/or generated synthetic spectra based on at least one of a similarity score, a matching probability, and a prediction from a machine learning model.

As previously discussed, processing toolchains used by database search engines that process raw spectrum files of the second set of raw spectrum files may be the same as or different from processing toolchains used by database search engines that process raw spectrum files of the first set of raw spectrum files. In some examples, even when the toolchains are the same, database search engines that process the second set of raw spectrum files may apply different criteria than database search engines that process the first set of raw spectrum files. For example, database search engines that process the second set of raw spectrum files may require less-exact matches between spectra in the raw spectrum file and reference library spectra and/or generated synthetic spectra (such as requiring fewer matching fragments, allowing for a higher mass deviation, and/or allowing for a higher deviation in retention time and/or other physico-chemical properties) then database search engines that process the first set of raw spectrum files.

In various implementations, the database search engines used to process the second set of raw spectrum files (such as database search engine 15002-2 and database search engine 15002-3) include or call upon further processing logic 17008 and/or quality control logic 17010 before generating result files for the second set of raw spectrum files (such as result files 14008-5-14008-4). In some examples, further processing logic 17008 may calculate a quantitation value. The quantitation value may be calculated (i) based on relative intensities within the sample and/or across samples, (ii) from signal intensities and/or spectral contribution factors as an area across multiple neighboring mass spectra, and/or (iii) using labeled or unlabeled calibration substances. In examples where quantitation values are calculated using labeled calibration substances, the labels may include mass tags and/or isotopic labels. In various implementations, further processing logic 17007 may determine and/or compare occurrences and/or quantitation comparisons across (i) the set of mass spectrometry data, the first subset of the mass spectrometry data, the second subset of the mass spectrometry data, further subsets of the mass spectrometry data, and/or a subset that includes the first subset and one or more additional elements of the set of mass spectrometry data.

In some embodiments, quality control logic 17010 may perform functions previously described with reference to quality control logic 17004. In some implementations, the results files output by the database search engines after processing the second batch of raw spectrum files may include databases of identifications and quantitations across the complete set of mass spectrometry data. In various implementations, the database search engines may provide - as an intermediate output - the subset of the set of mass spectrometry data processed so far. In various implementations, the contents of the outputs - such as contents of the result files - are presented via a graphical user interface output to a screen. The output may be interrogated by a user or other data system to determine significant differences between samples and/or the presence or absence of certain substances from one or more samples.

As illustrated in FIGS. 14-17, the implementation of the second process 3000 provides computational advantages over the implementation of the first process 2000 as illustrated in FIG. 13. For example, as previously discussed, processes implemented according to FIG. 13 require the entire batch of *n* raw spectrum files to be processed by machine learning model 13004 and the entire batch *n* of initial spectrum match files to be processed by analysis logic 1006 before a screening list is generated. This requires 2×*n* computational operations before the screening list is generated. By contrast, processes implemented according to FIGS. 14-16 (such as process 3000) require only the subset of *m* raw spectrum files to be processed by machine learning model 13004 and the subset of m initial spectrum match files to be processed by analysis logic 1006 before the screening list (or database of identified entities) is generated - requiring only 2×*m* computational operations. In examples where m is 10% of *n,* processes implemented according to FIGS. 14-17 require only 10% of the computational time of processes implemented according to FIG. 13 - providing up to a tenfold increase in computational throughput. This increase in computational throughput allows processes implemented according to FIGS. 14-17 to be completed in real time or near-real time (e.g., provide results for each raw spectrum file as such a file is generated).

Furthermore, various implementations of processes implemented according to FIGS. 14-17 can generate a screening list and/or database of identified entities before complete results for the entire batch of n raw spectrum files are generated. For example, the screening list and/or database of identified entities may be generated after only a single raw spectrum file from the first subset is processed, so the support module 1000 can immediately begin processing next samples - such as raw spectrum files of the second subset - in real-time or near-real-time as they are generated by the scientific instrument 12010.

The following paragraphs provide various examples of the embodiments disclosed herein.

Example 1 includes a scientific instrument support apparatus including memory hardware configured to store instructions and processing hardware configured to execute the instructions. The instructions include loading a batch of raw spectrum files generated by a mass spectrometer, dividing the raw spectrum files into a first subset and a second subset, processing each of the first subset of raw spectrum files with a machine learning model to generate a first subset of spectrum match files, generating a screening list from the first subset of spectrum match files, and processing each of the second subset of raw spectrum files and the screening list with the machine learning model to generate a second subset of spectrum match files.

Example 2 includes the subject matter of Example 1, and the instructions further include generating a results list from the second subset of spectrum match files.

Example 3 includes the subject matter of Example 1, and the instructions further include processing each of the first subset of raw spectrum files and the screening list with the machine learning model to generate an updated first subset of spectrum match files and generating a results list from the updated first subset of spectrum match files and the second subset of spectrum match files.

Example 4 includes the subject matter of any of Examples 1-3 and further specifies the machine learning model is configured to generate each spectrum match file by preprocessing a selected raw spectrum file, loading a protein database, generating a test spectrum for each peptide in the protein database, and matching spectra in the preprocessed spectrum file with the generated test spectra and generating a score evaluating a closeness of each match.

Example 5 includes the subject matter of Example 4 and further specifies the machine learning model is configured to generate each spectrum file by determining whether the screening list is loaded and in response to determining that the screening list is not loaded: (i) discarding matched spectra having scores below a first threshold and (ii) saving remaining matched spectra to the spectrum match file.

Example 6 includes the subject matter of Example 4 and further specifies that the machine learning model is configured to generate each spectrum file by determining whether the screening list is loaded. In response to determining that the screening list is loaded, the machine learning model is configured to determine whether the screening list includes an inclusion list, discard matched spectra having scores below a first threshold and that are not on the inclusion list, determine whether the screening list includes an exclusion list, and discard matched spectra on the exclusion list in response to determining that the screening list includes the exclusion list. The machine learning model is configured to discard matched spectra having scores below the first threshold and save remaining matched spectra to the spectrum match file.

Example 7 includes the subject matter of any of Examples 1-6 and further specifies that generating the screening list from the first subset of spectrum match files includes parsing the first subset of spectrum match files to identify peptides present, calculating a frequency of appearance for each of the identified peptides, discarding identified peptides having a frequency of appearance below a second threshold; and adding the remaining identified peptides to an inclusion list.

Example 8 includes the subject matter of any of Examples 1-7 and further specifies that the screening list from the first subset of spectrum match files includes generating filtered spectrums by removing peaks below an intensity threshold from spectrums of the first subset of spectrum match files, processing the filtered spectrums to identify peptides associated with the filtered spectrums, counting a number of occurrences of each identified peptide, and saving peptides having a number of occurrences below a third threshold to the exclusion list.

Example 9 includes the subject matter of Example 4 wherein preprocessing the selected raw spectrum file includes detecting peaks in a spectrum of the raw spectrum file, removing noise from the spectrum, applying a baseline correction to the spectrum, applying mass calibration to the spectrum, and applying deconvolution processing to the spectrum.

Example 10 includes the subject matter of Examples 1-9 wherein the mass spectrometer generates raw spectrum files by ionizing a prepared sample, performing ion separation on the ionized sample, detecting separated ions, and generating a mass spectrum from the detected separated ions.

Example 11 includes computer-implemented method for scientific instrument support that includes loading a batch of raw spectrum files generated by a mass spectrometer, dividing the raw spectrum files into a first subset and a second subset, processing each of the first subset of raw spectrum files with a machine learning model to generate a first subset of spectrum match files, generating a screening list from the first subset of spectrum match files, and processing each of the second subset of raw spectrum files and the screening list with the machine learning model to generate a second subset of spectrum match files.

Example 12 includes the subject matter of Example 11 and further specifies generating a results list from the second subset of spectrum match files.

Example 13 includes the subject matter of Example 11 and further specifies processing each of the first subset of raw spectrum files and the screening list with the machine learning model to generate an updated first subset of spectrum match files and generating a results list from the updated first subset of spectrum match files and the second subset of spectrum match files.

Example 14 includes the subject matter of any of Examples 11-13 and further specifies that the machine learning model is configured to generate each spectrum match file by preprocessing a selected raw spectrum file, loading a protein database, generating a test spectrum for each peptide in the protein database, and matching spectra in the preprocessed spectrum file with the generated test spectra and generating a score evaluating a closeness of each match.

Example 15 includes the subject matter of Example 14 and further specifies that the machine learning model is configured to generate each spectrum file by determining whether the screening list is loaded; and in response to determining that the screening list is not loaded: (i) discarding matched spectra having scores below a first threshold and (ii) saving remaining matched spectra to the spectrum match file.

Example 16 includes the subject matter of Example 14 and further specifies that the machine learning model is configured to generate each spectrum file by determining whether the screening list is loaded In response to determining that the screening list is loaded, the machine learning model is configured to generate each spectrum file by determining whether the screening list includes an inclusion list, discarding matched spectra having scores below a first threshold and that are not on the inclusion list in response to determining that the screening list includes the inclusion list, determining whether the screening list includes an exclusion list, and discarding matched spectra on the exclusion list in response to determining that the screening list includes the exclusion list. The machine learning model is configured to generate each spectrum file by discarding matched spectra having scores below the first threshold and saving remaining matched spectra to the spectrum match file.

Example 17 includes the subject matter of any of Examples 11-16 and further specifies that generating the screening list from the first subset of spectrum match files includes parsing the first subset of spectrum match files to identify peptides present, calculating a frequency of appearance for each of the identified peptides, discarding identified peptides having a frequency of appearance below a second threshold, and adding the remaining identified peptides to an inclusion list.

Example 18 includes the subject matter of any of Examples 11-17 and further specifies that generating the screening list from the first subset of spectrum match files includes generating filtered spectrums by removing peaks below an intensity threshold from spectrums of the first subset of spectrum match files, processing the filtered spectrums to identify peptides associated with the filtered spectrums, counting a number of occurrences of each identified peptide, and saving peptides having a number of occurrences below a third threshold to the exclusion list.

Example 19 includes the subject matter of Example 14 and further specifies that preprocessing the selected raw spectrum file includes detecting peaks in a spectrum of the raw spectrum file, removing noise from the spectrum, applying a baseline correction to the spectrum, applying mass calibration to the spectrum, and applying deconvolution processing to the spectrum.

Example 20 incudes the subject matter of Examples 11-19 and further specifies that the mass spectrometer generates raw spectrum files by ionizing a prepared sample, performing ion separation on the ionized sample, detecting separated ions, and generating a mass spectrum from the detected separated ions.

Example 21 includes a scientific instrument support apparatus that includes first logic to receive a batch of raw data structures generated by a mass spectrometer and second logic to divide the batch of raw data structures into a first subset and a second subset, generate a first subset of processed data structures by providing each of the first subset of raw data structures to an artificial-intelligence-enabled data analysis system, parse the first subset of processed data structures to build a comparison list, and generate a second subset of processed data structures by providing each of the second subset of raw data structures and the comparison list to the artificial-intelligence-enabled data analysis system.

Example 22 includes the subject matter of Example 21 and further specifies that the mass spectrometer is configured to generate the raw data structures by ionizing a prepared sample, performing ion separation on the ionized sample, detecting separated ions, and generating a mass spectrum from the detected separated ions.

Example 23 includes the subject matter of Examples 21-22 and further specifies that the artificial-intelligence-enabled data analysis system is configured to preprocess a selected data structure, load a database, generate a test spectrum for each peptide in the database, and match spectra in the preprocessed data structure with the generated test spectra and generate a score evaluating a closeness of each match.

Example 24 includes the subject matter of Example 23 and further specifies that the artificial-intelligence-enabled data analysis system is configured to determine whether the comparison list is loaded and, in response to determining that the comparison list is not loaded: discard matched spectra having scores below a first threshold and save remaining matched spectra to the processed data structure.

Example 25 includes the subject matter of Example 23 and further specifies that the artificial-intelligence-enabled data analysis system is configured to determine whether the comparison list is loaded. In response to determining that the comparison list is loaded, the artificial-intelligence-enabled data analysis system is configured to determine whether the comparison list includes an inclusion list, discard matched spectra having scores below a first threshold and that are not on the inclusion list in response to determining that the comparison list includes the inclusion list, determine whether the comparison list includes an exclusion list, and discard matched spectra on the exclusion list in response to determining that the comparison list includes the exclusion list. The artificial-intelligence-enabled data analysis system is configured to discard matched spectra having scores below the first threshold and save remaining matched spectra to the processed data structure.

Example 26 includes the subject matter of any of Examples 23-25 and further specifies that preprocessing the selected data structure includes detecting peaks in a spectrum of the selected data structure, removing noise from the spectrum, applying a baseline correction to the spectrum, applying mass calibration to the spectrum, and applying deconvolution processing to the spectrum.

Example 27 includes the subject matter of any of Examples 21-26 and further specifies that the second logic is configured to build the comparison list by parsing the first subset of processed data structures to identify peptides present, calculating a frequency of appearance for each of the identified peptides, discarding identified peptides having a frequency of appearance below a second threshold, and adding the remaining identified peptides to an inclusion list.

Example 28 includes the subject matter of any of Examples 21-27 and further specifies that the second logic is configured to build the comparison list by parsing the first subset of processed data structures to generate filtered spectrums by removing peaks below an intensity threshold, processing the filtered spectrums to identify peptides associated with the filtered spectrums, counting a number of occurrences of each identified peptide, and saving peptides having a number of occurrences below a third threshold to the exclusion list.

Example 29 includes the subject matter of any of Examples 21-28 and further specifies that the second logic is configured to generate an output list by processing the second subset of processed data structures.

Example 30 includes the subject matter of any of Examples 21-28 and further specifies that the second logic is configured to generate an updated first subset of processed data structures by providing each of the first subset of raw data structures and the comparison list to the artificial-intelligence-enabled data analysis system and generate an output list by processing the updated first subset of processed data structures and the second subset of processed data structures.

Example 31 includes a method for scientific instrument support that includes loading a batch of raw data structures generated by a mass spectrometer, dividing the batch of raw data structures into a first subset and a second subset, generating a first subset of processed data structures by providing each of the first subset of raw data structures to an artificial-intelligence-enabled data analysis system, parsing the first subset of processed data structures to build a comparison list, and generating a second subset of processed data structures by providing each of the second subset of raw data structures and the comparison list to the artificial-intelligence-enabled data analysis system.

Example 32 includes the subject matter of Example 31 and further specifies that the mass spectrometer is configured to generate the raw data structures by ionizing a prepared sample, performing ion separation on the ionized sample, detecting separated ions, and generating a mass spectrum from the detected separated ions.

Example 33 includes the subject matter of any of Examples 31-32 and further specifies that the artificial-intelligence-enabled data analysis system is configured to preprocess a selected data structure, load a database, generate a test spectrum for each peptide in the database, and match spectra in the preprocessed data structure with the generated test spectra and generate a score evaluating a closeness of each match.

Example 34 includes the subject matter of Example 33 and further specifies that the artificial-intelligence-enabled data analysis system is configured to determine whether the comparison list is loaded and in response to determining that the comparison list is not loaded: discarding matched spectra having scores below a first threshold and saving remaining matched spectra to the processed data structure.

Example 35 includes the subject matter of Example 33 and further specifies that the artificial-intelligence-enabled data analysis system is configured to determine whether the comparison list is loaded. In response to determining that the comparison list is loaded, the artificial-intelligence-enabled data analysis system is configured to determine whether the comparison list includes an inclusion list, discard matched spectra having scores below a first threshold and that are not on the inclusion list in response to determining that the comparison list includes the inclusion list, determine whether the comparison list includes an exclusion list, and discard matched spectra on the exclusion list in response to determining that the comparison list includes the exclusion list. The artificial-intelligence-enabled data analysis system is configured to discard matched spectra having scores below the first threshold and save remaining matched spectra to the processed data structure.

Example 36 includes the subject matter of any of Examples 33-35 and further specifies that preprocessing the selected data structure includes detecting peaks in a spectrum of the selected data structure, removing noise from the spectrum, applying a baseline correction to the spectrum, applying mass calibration to the spectrum, and applying deconvolution processing to the spectrum.

Example 37 includes the subject matter of any of Examples 31-36 and further specifies that parsing the first subset of processed data structures to build the comparison list includes parsing the first subset of processed data structures to identify peptides present, calculating a frequency of appearance for each of the identified peptides, discarding identified peptides having a frequency of appearance below a second threshold, and adding the remaining identified peptides to an inclusion list.

Example 38 includes the subject matter of any of Examples 31-37 and further specifies that parsing the first subset of processed data structures to build the comparison list includes parsing the first subset of processed data structures to generate filtered spectrums by removing peaks below an intensity threshold, processing the filtered spectrums to identify peptides associated with the filtered spectrums, counting a number of occurrences of each identified peptide, and saving peptides having a number of occurrences below a third threshold to the exclusion list.

Example 39 includes the subject matter of any of Examples 31-38 and further specifies generating an output list by processing the second subset of processed data structures.

Example 40 includes the subject matter of any of Examples 31-38 and further specifies generating an updated first subset of processed data structures by providing each of the first subset of raw data structures and the comparison list to the artificial-intelligence-enabled data analysis system and generating an output list by processing the updated first subset of processed data structures and the second subset of processed data structures,

Example 41 includes a method for scientific instrument support that includes receiving a first set of mass spectrometry data, processing the first set of mass spectrometry data to generate a database of identified entities, receiving a second set of mass spectrometry data, and processing the second set of mass spectrometry data to identify and/or quantitate entities based on the database of identified entities.

Example 42 includes the subject matter of Example 41 and further specifies that the first set of mass spectrometry data and the second set of mass spectrometry data are generated using a same data acquisition method.

Example 43 includes the subject matter of Example 42 and further specifies that the data acquisition method is a data independent acquisition method.

Example 44 includes the subject matter of Example 42 and further specifies that the data acquisition method is a data dependent acquisition method.

Example 45 includes the subject matter of any of Examples 41-44 and further specifies that processing the first set of mass spectrometry data to generate the database of identified entities includes comparing ion spectra from the first set of mass spectrometry data to a reference database.

Example 46 includes the subject matter of any of Examples 41-45 and further specifies that processing the first set of mass spectrometry data to generate the database of identified entities includes adding entities from the first set of mass spectrometry data that meet a minimum quality criterion to the database of identified entities.

Example 47 includes the subject matter of Example 46 and further specifies that the minimum quality criterion is set according to at least one of a threshold, false detection rate, or spectral match score.

Example 48 includes the subject matter of any of Examples 41-47 and further specifies that the database of identified entities includes peptide sequences.

Example 49 includes the subject matter of any of Examples 41-48 and further specifies that the database of identified entities includes peptide identifications.

Example 50 includes the subject matter of any of Examples 41-49 and further specifies that the database of identified entities includes mass spectra.

Example 51 includes the subject matter of any of Examples 41-50 and further specifies that the database of identified entities includes precursor ion information.

Example 52 includes the subject matter of Example 51 and further specifies that the precursor ion information includes mass information.

Example 53 includes the subject matter of any of Examples 51-52 and further specifies that the precursor ion information includes mass-to-charge ratios.

Example 54 includes the subject matter of any of Examples 51-53 and further specifies that the precursor ion information includes mass-to-charge windows.

Example 55 includes the subject matter of any of Examples 41-54 and further specifies processing the first set of mass spectrometry data to identify and/or quantitate entities based on the database of identified entities.

Example 56 includes the subject matter of any of Examples 41-55 and further specifies processing the second set of mass spectrometry data to identify and/or quantitate entities based on the database of identified entities includes comparing ion spectra from the second set of mass spectrometry data with entries in the database of identified entities.

Example 57 includes the subject matter of any of Examples 41-56 and further specifies processing the second set of mass spectrometry data to identify and/or quantitate entities based on the database of identified entities includes comparing fragmentation spectra from the second set of mass spectrometry data with entries in the database of identified entities.

Example 58 includes the subject matter of any of Examples 41-56 and further specifies processing the second set of mass spectrometry data to identify and/or quantitate entities based on the database of identified entities includes searching the second set of mass spectrometry data for entities in the database of identified entities.

Example 59 includes the subject matter of Example 58 and further specifies searching the second set of mass spectrometry data for entities in the database of identified entities includes searching the database of identified entities for at least one of precursor information or retention time information.

Example 60 includes the subject matter of any of Examples 41-59 and further specifies processing at least some of the second set of mass spectrometry data to extend the database of identified entities.

Example 61 includes the subject matter of Example 60 and further specifies processing at least some of the second set of mass spectrometry data to extend the database of identified entities includes re-searching already processed members of the first and second sets of mass spectrometry data to receive further identification and/or quantification information.

Example 62 includes the subject matter of Example 61 and further specifies processing at least some of the second set of mass spectrometry data to extend the database of identified entities is stopped in response to a growth rate of the database of identified entities falling below a second threshold.

Example 63 includes the subject matter of Example 62 and further specifies that the second threshold is an average of less than 10 addition entries per member of the second set of mass spectrometry data.

Example 64 includes the subject matter of Example 62 and further specifies that the second threshold is an average of less than 1 addition entries per member of the second set of mass spectrometry data.

Example 65 includes the subject matter of Example 62 and further specifies that the second threshold is an average of less than 0.1 addition entries per member of the second set of mass spectrometry data.

Example 66 includes the subject matter of Example 62 and further specifies that the second threshold is an average of less than 0.01 addition entries per member of the second set of mass spectrometry data.

Example 67 includes the subject matter of any of Examples 41-66 and further specifies that members of the first set of mass spectrometry data is selected to have a higher concentration than members of the second set of mass spectrometry data.

Example 68 includes scientific instrument support apparatus that includes memory hardware configured to store instructions and processing hardware configured to execute the instructions, which when executed by the processing hardware causes the scientific instrument support apparatus to perform the method of any of Examples 41-67.

Example 69 includes one or more non-transitory computer-readable media having instructions thereon that, when executed by one or more processing devices of a scientific instrument support apparatus, cause the scientific instrument support apparatus to perform the method of any of Examples 11-20.

Example 70 includes one or more non-transitory computer-readable media having instructions thereon that, when executed by one or more processing devices of a scientific instrument support apparatus, cause the scientific instrument support apparatus to perform the method of any of Examples 31-40.

Example 71 includes one or more non-transitory computer-readable media having instructions thereon that, when executed by one or more processing devices of a scientific instrument support apparatus, cause the scientific instrument support apparatus to perform the method of any of Examples 41-66.

Example 72 includes a method for scientific instrument support that includes receiving a first set of mass spectrometry files representing one or more samples, analyzing each spectrum file of the first set of mass spectrometry data with a selected machine learning model from a first set of machine learning models to generate initial results, analyzing the initial results to generate a screening list, receiving one or more raw spectrum files from a second set of mass spectrometry data, analyzing each of the one or more raw spectrum files from the second set of mass spectrometry data at a selected machine learning model from a second set of machine learning models to generate result files, and saving the result files to a data store.

Example 73 includes the subject matter of Example 72 and further specifies that the selected machine learning model from the first set of machine learning models is the same as the selected machine learning model from the second set of machine learning models.

Example 74 includes the subject matter of Example 72 and further specifies that the selected machine learning model from the first set of machine learning models is different from the selected machine learning model from the second set of machine learning models.

Example 75 includes the subject matter of any of Examples 72-74 and further specifies that the selected machine learning model from the first set of machine learning models and the selected machine learning model from the second set of machine learning models includes a database search engine.

Example 76 includes the subject matter of Example 75 and further specifies that the database search engine is a peptide search engine.

Example 77 includes the subject matter of any of Examples 72-76 and further specifies analyzing the initial results to generate the screening list includes merging high-confidence identifications from all searches into one screening list of identified entities for a given experimental setup.

Example 78 includes a scientific instrument support apparatus that includes memory hardware configured to store instructions and processing hardware configured to execute the instructions, which when executed by the processing hardware causes the scientific instrument support apparatus to perform the method of any of Examples 72-77.

Example 79 includes one or more non-transitory computer-readable media having instructions thereon that, when executed by one or more processing devices of a scientific instrument support apparatus, cause the scientific instrument support apparatus to perform the method of any of Examples 72-77.

Example 80 includes method for scientific instrument support that includes receiving a first subset of a set of mass spectrometry data, receiving a first screening list, processing the first subset of mass spectrometry data and the first screening list at a first database search engine to generate a second screening list, receiving a second subset of the set of mass spectrometry data, and providing each file of the second subset of mass spectrometry data and a target screening list to a second database search engine to generate a result file for each file of the second subset of mass spectrometry data. The target screening list being based on the second screening list.

Example 81 includes the subject matter of Example 80 and further specifies that the second screening list is provided to the second database search engine as the target screening list.

Example 82 includes the subject matter of Example 80 and further specifies that the target screening list is generated by merging the first screening list and the second screening list.

Example 83 includes the subject matter of any of Examples 80-82 and further specifies that the set of mass spectrometry data includes data from one or more connected studies.

Example 84 includes the subject matter of Example 83 and further specifies that the set of mass spectrometry data includes at least one of mass data, intensity data, a retention time, ion mobility data, a physico-chemical property, and a location on a spatially arranged sample.

Example 85 includes the subject matter of any of Examples 80-84 and further specifies that elements of the set of mass spectrometry data are related by at least one of a similarity of samples and a similarity of data acquisition methods.

Example 86 includes the subject matter of any of Examples 80-85 and further specifies that the first screening list is formatted in a FASTA format.

Example 87 includes the subject matter of any of Examples 80-86 and further specifies that processing the first subset of mass spectrometry data and the first screening list at the first database search engine to generate the second screening list includes selecting entities according to criteria.

Example 88 includes the subject matter of Example 87 and further specifies that the entities include proteins or peptides.

Example 89 includes the subject matter of any of Examples 87-88 and further specifies that selecting entities according to criteria includes determining that each entities passes or fails a quality control test and, in response to determining that each entity passes the quality control test, adding the entity to a database of identified entities.

Example 90 includes the subject matter of Example 89 and further specifies that the quality control test includes at least one of selecting entities based on a false discovery rate, determining whether entities meet or exceed a spectral quality threshold, determining whether entities have at least a number of peaks in common with a reference, and determining whether entities meet or exceed a minimum number of occurrences in the subset.

Example 91 includes the subject matter of Example 89 and further specifies that the quality control test includes ranking entities according to a percolator machine learning model and separating true positive entity identifications from incorrect entity identifications.

Example 92 includes the subject matter of any of Examples 87-91 and further specifies that each entity is represented by at least one of an entity identifier, a protein sequence, a peptide sequence, one or more masses from a mass spectrometry (MS) spectrometer, one or more masses from a tandem mass spectrometry (MS/MS) spectrometer, an intensity value, a physico-chemical property, a retention time, or an ion mobility.

Example 93 includes the subject matter of any of Examples 80-92 and further specifies that wherein providing each file of the second subset of mass spectrometry data and the target screening list to the second database search engine to generate the result file for each file of the second subset of mass spectrometry data includes at least one of excluding any entities not present in the target screening list from further processing and including any entities present in the target screening list for further processing.

Example 94 includes the subject matter of any of Examples 80-93 and further specifies that providing each file of the second subset of mass spectrometry data and the target screening list to the second database search engine to generate the result file for each file of the second subset of mass spectrometry data includes comparing mass spectrometry data from each file of the second subset to library spectra data.

Example 95 includes the subject matter of any of Examples 80-93 and further specifies that providing each file of the second subset of mass spectrometry data and the target screening list to the second database search engine to generate the result file for each file of the second subset of mass spectrometry data includes mass spectrometry data from each file of the second subset to synthetic spectra created based on entities present in the target screening list.

Example 96 includes the subject matter of any of Examples 94-95 and further specifies that mass spectrometry data from each file of the second subset includes at least one of mass data, intensity data, retention time data, and ion mobility data.

Example 97 includes the subject matter of any of Examples 80-96 and further specifies that the first database search engine and the second database search engine apply same processing toolchains.

Example 98 includes the subject matter of any of Examples 80-96 and further specifies that the first database search engine and the second database search engine apply different processing toolchains.

Example 99 includes the subject matter of any of Examples 80-98 and further specifies that the first database search engine matches entities from the first subset of mass spectrometry data with first reference entities based on a first criterion, the second database search engine matches entities from the second subset of mass spectrometry data with second reference entities based on a second criterion, and the first criterion requires a greater match than the second criterion.

Example 100 includes the subject matter of Example 99 and further specifies that the first criterion includes matching entities based on at least one of fragments, mass deviation, retention time, and physico-chemical properties.

Example 101 includes the subject matter of Examples 99-100 and further specifies that the second criterion includes matching entities based on at least one of fragments, mass deviation, retention time, and physico-chemical properties.

Example 102 includes the subject matter of any of Examples 80-101 and further specifies that the second database search engine is configured to output an aligned database of identifications per sample.

Example 103 includes the subject matter of any of Examples 80-102 and further specifies that the second database search engine is configured to perform further processing steps by calculating a quantitation value.

Example 104 includes the subject matter of Example 103 and further specifies that the second database search engine is configured to calculate the quantitation value based on relative intensities within a sample.

Example 105 includes the subject matter of Example 103 and further specifies that the second database search engine is configured to calculate the quantitation value based on relative intensities across samples.

Example 106 includes the subject matter of Example 103 and further specifies that the second database search engine is configured to calculate the quantitation value from signal intensities across multiple neighboring mass spectra.

Example 107 includes the subject matter of Example 103 and further specifies that the second database search engine is configured to calculate the quantitation value from spectral contribution factors across multiple neighboring mass spectra.

Example 108 includes the subject matter of Example 103 and further specifies that the second database search engine is configured to calculate the quantitation value using unlabeled calibration substances.

Example 109 includes the subject matter of Example 103 and further specifies that the second database search engine is configured to calculate the quantitation value using labeled calibration substances.

Example 110 includes the subject matter of Example 109 and further specifies that labels of the labeled calibration substances include at least one of mass tags and isotopic labels.

Example 111 includes the subject matter of any of Examples 102-110 and further specifies that the second database search engine is configured to determine occurrences across at least one of the set of mass spectrometry data, the first subset of the mass spectrometry data, the second subset of the mass spectrometry data, further subsets of the mass spectrometry data, and a third subset including the first subset and one or more additional elements of the set of mass spectrometry data.

Example 112 includes the subject matter of any of Examples 102-110 and further specifies that the second database search engine is configured to compare occurrences across at least one of the set of mass spectrometry data, the first subset of the mass spectrometry data, the second subset of the mass spectrometry data, further subsets of the mass spectrometry data, and a third subset including the first subset and one or more additional elements of the set of mass spectrometry data.

Example 113 includes the subject matter of any of Examples 102-110 and further specifies that the second database search engine is configured to determine quantitation comparisons across at least one of the set of mass spectrometry data, the first subset of the mass spectrometry data, the second subset of the mass spectrometry data, further subsets of the mass spectrometry data, and a third subset including the first subset and one or more additional elements of the set of mass spectrometry data.

Example 114 includes the subject matter of any of Examples 102-113 and further specifies that the second database search engine is configured to output a database of identifications and quantitations across the set of mass spectrometry data.

Example 115 includes the subject matter of any of Examples 102-113 and further specifies that the second database search engine is configured to output a database of identifications and quantitations across a portion of set of mass spectrometry data.

Example 116 includes the subject matter of any of Examples 80-115 and further specifies outputting the at least one result file to a graphical user interface displayed on a screen, wherein the graphical user interface is configured to allow a user or other data system to interrogate the at least one result file for at least one of: (i) significant differences between samples, (ii) a presence of substances within one or more samples, and (iii) an absence of substances within one or more samples.

Example 117 includes a scientific instrument support apparatus that includes memory hardware configured to store instructions and processing hardware configured to execute the instructions, which when executed by the processing hardware causes the scientific instrument support apparatus to perform the method of any of Examples 80-116.

Example 118 includes one or more non-transitory computer-readable media having instructions thereon that, when executed by one or more processing devices of a scientific instrument support apparatus, cause the scientific instrument support apparatus to perform the method of any of Examples 72-116.

## Claims

1. A computer-implemented method for scientific instrument support including:
loading a batch of raw spectrum files generated by a mass spectrometer;
dividing the raw spectrum files into a first subset and a second subset;
processing each of the first subset of raw spectrum files with a machine learning model to generate a first subset of spectrum match files;
generating a screening list from the first subset of spectrum match files; and
processing each of the second subset of raw spectrum files and the screening list with the machine learning model to generate a second subset of spectrum match files.

2. The method of claim 1 including generating a results list from the second subset of spectrum match files.

3. The method of claim 1 including:
processing each of the first subset of raw spectrum files and the screening list with the machine learning model to generate an updated first subset of spectrum match files; and
generating a results list from the updated first subset of spectrum match files and the second subset of spectrum match files.

4. The method of claim 1, wherein the machine learning model is configured to generate each spectrum match file by:
preprocessing a selected raw spectrum file;
loading a protein database;
generating a test spectrum for each peptide in the protein database; and
matching spectra in the preprocessed spectrum file with the generated test spectra and generating a score evaluating a closeness of each match.

5. The method of claim 4, wherein the machine learning model is configured to generate each spectrum file by:
determining whether the screening list is loaded; and
in response to determining that the screening list is not loaded:
discarding matched spectra having scores below a first threshold, and
saving remaining matched spectra to the spectrum match file.

6. The method of claim 4, wherein the machine learning model is configured to generate each spectrum file by:
determining whether the screening list is loaded;
in response to determining that the screening list is loaded:
determining whether the screening list includes an inclusion list,
in response to determining that the screening list includes the inclusion list:
discarding matched spectra having scores below a first threshold and that are not on the inclusion list,
determining whether the screening list includes an exclusion list,
in response to determining that the screening list includes the exclusion list:
discarding matched spectra on the exclusion list;
discarding matched spectra having scores below the first threshold; and
saving remaining matched spectra to the spectrum match file.

7. The method of claim 6, wherein generating the screening list from the first subset of spectrum match files includes:
parsing the first subset of spectrum match files to identify peptides present;
calculating a frequency of appearance for each of the identified peptides;
discarding identified peptides having a frequency of appearance below a second threshold; and
adding the remaining identified peptides to an inclusion list.

8. The method of claim 7, wherein generating the screening list from the first subset of spectrum match files includes:
generating filtered spectrums by removing peaks below an intensity threshold from spectrums of the first subset of spectrum match files;
processing the filtered spectrums to identify peptides associated with the filtered spectrums;
counting a number of occurrences of each identified peptide; and
saving peptides having a number of occurrences below a third threshold to the exclusion list.

9. A scientific instrument support apparatus including memory hardware and processing hardware, the memory hardware being configured to store instructions which, when executed by the processing hardware, carry out the method steps of any one of the preceding claims.
